# EUROPEAN PATENT SPECIFICATION

(11) **EP 2 636 753 B1**
(45) Date of publication and mention of the grant of the patent: **25.07.2018**
(21) Application number: 13156208.4
(22) Date of filing: 21.02.2013
(51) Int. Cl.: C12Q 1/68

(54) **Method and means for identification of animal species**
Verfahren und Mittel zur Identifizierung von Tierarten
Procédé et moyen pour l'identification d'espèces animales

(30) Priority: 09.03.2012 US 201261608824 P
(43) Date of publication of application: 11.09.2013
(73) Proprietor: City University of Hong Kong, Hong Kong (CN)
(72) Inventor: Cheng, Shuk Han, Hong Kong (CN); Lin, Chun Chi, Hong Kong (CN)
(74) Representative: Reininger, Jan Christian

(56) References cited:
- WO-A2-03/048723
- WO-A2-2008/056325
- JP-A- 2009 195 226
- US-A1- 2006 019 295
- MEUSNIER ISABELLE ET AL: "A universal DNA mini-barcode for biodiversity analysis", BMC GENOMICS, BIOMED CENTRAL LTD, LONDON, UK, vol. 9, no. 1, 12 May 2008 (2008-05-12), page 214, XP021032945, ISSN: 1471-2164
- LOCKLEY A ET AL: "DNA-based methods for food authentication", TRENDS IN FOOD SCIENCE AND TECHNOLOGY, ELSEVIER SCIENCE PUBLISHERS, GB, vol. 11, no. 2, 1 February 2000 (2000-02-01), pages 67-77, XP027357258, ISSN: 0924-2244 [retrieved on 2000-02-01]
- SHADI SHOKRALLA ET AL: "Pyrosequencing for Mini-Barcoding of Fresh and Old Museum Specimens", PLOS ONE, vol. 6, no. 7, 27 July 2011 (2011-07-27), page e21252, XP055064962, DOI: 10.1371/journal.pone.0021252
- HANDY SARA M ET AL: "A Single-Laboratory Validated Method for the Generation of DNA Barcodes for the Identification of Fish for Regulatory Compliance", JOURNAL OF AOAC INTERNATIONAL, vol. 94, no. 1, January 2011 (2011-01), pages 201-210, XP009169980, ISSN: 1060-3271
- HAJIBABAEI MEHRDAD ET AL: "Design and applicability of DNA arrays and DNA barcodes in biodiversity monitoring", BMC BIOLOGY, BIOMED CENTRAL, LONDON, GB, GB, vol. 5, no. 1, 13 June 2007 (2007-06-13), page 24, XP021030215, ISSN: 1741-7007, DOI: 10.1186/1741-7007-5-24
- POZHITKOV ALEXANDER E ET AL: "An algorithm and program for finding sequence specific oligo-nucleotide probes for species identification", BMC BIOINFORMATICS, BIOMED CENTRAL, LONDON, GB, vol. 3, no. 1, 6 March 2002 (2002-03-06), page 9, XP021013576, ISSN: 1471-2105, DOI: 10.1186/1471-2105-3-9

## Description

### FIELD OF THE INVENTION

The present invention is concerned with a method of identification of species, and in particular a method of making relatively rapid identification of animal species including but not limited to *Bos Taurus* (cattle), *Sus scrofa* (pig), *Ovis aries* (sheep), *Equus caballus* (horse), *Canis lupus* (dog) and *Mus musculus* (mouse) and *Felis Catus* (cat). The present invention is also concerned with means for use in carrying out such method.

### BACKGROUND OF THE PRESENT INVENTION

Due to a vast range of reasons, there has been a need of identifying animal species origin of a sample. For example, in the context of forensic investigation there is often the need of ascertaining the identity of an animal tissue sample in a crime scene. In the context of quality control of food manufacturing, there is a need to ascertain whether a food product claimed to contain certain type of animal meat is indeed what it claims to be. There are actually many other possible applications of using animal species origin or animal tissue identification methods, and one can also envisage that such applications can be very useful in medical science and pharmaceutical industry, and of course to address food safety issues as well.

There have been many methodologies seeking to address the needs of identification of animal species. While many of these methodologies have their unique characteristics, they often suffer from drawbacks, and tend to address some issues but not others. For example, some methodologies tend to be fairly accurate in their identification but at the expense of tremendous complexity in procedures. Some other methodologies are relatively simple in terms of procedures but often involve using high end equipment which requires significant financial investment and specially trained personnel (e.g. high speed DNA sequencing). Yet some methodologies are easy to grasp but the time needed to conduct identification is too long and commercially unrealistic and thus virtually unusable. Yet further, some methodologies are not able to distinguish a specimen when the sample contains multiple sources of animal species tissues, or at least they are not able to distinguish a specimen in one experiment.

US2006/019295A1 relates to a method for identifying oligonucleotide probes for use as genetic tags in a genomic bar coding assay. The method comprises the steps of selecting a nucleotide sequence in a genome of a first organism and analyzing the whole genome of a second organism for the presence or the absence of that nucleotide sequence, and classifying that nucleotide sequence either as taxon-specific probe or homologous probe. This method is time-consuming and especially unrealistic, if there are multiple samples to be checked, not knowing whether all these samples are of different origin or whether analysis of these samples should be run against all sequence database.

Further, M. Hajibabaei al. in "Design and applicability of DNA arrays and DNA barcodes in biodiversity monitoring", BMC BIOLOGY, BIOMED CENTRAL, LONDON, GB, GB, vol. 5, no. 1, 13 June 2007, page 24, disclose methods for identification of species on the basis of DNA arrays and DNA barcodes. There is taught a division of the 650bp barcode into six 109bp mini barcodes and indicated that first 150bp from 5' end of COI and Cyb is used for probes designing. Three 25-mer probes of both COI and Cyb for each species are used.

A. E. Pozhitkov et al. in "An algorithm and program for finding sequence specific oligo-nucleotide probes for species identification", BMC BIOINFORMATICS, BIOMED CENTRAL, LONDON, GB, vol. 3, no. 1, 6 March 2002 , page 9, describe the use of an algorithm for probe selection.

The present invention seeks to address the aforementioned issues by providing a method which endeavors to balance different factors, or at least to provide an alternative balance, and means thereof. At least the present invention seeks to provide an alternative to the public for identifying an animal species sample, and means thereof.

### SUMMARY OF THE INVENTION

According to a first aspect of the present invention, there is provided a method of simultaneous identification of different mammalian species origins of a sample, comprising the steps of:
a) dividing the sample into a number of portions and situating the sample portions in a multi-well container or containers for use in DNA hybridization;
b) providing DNA probes of SEQ ID NOs. 1-241 with a length from 60-80 bases, wherein the number of the sample portions is greater than the number of mammalian species from which the DNA probes derive;
c) hybridizing the sample portions with the DNA probes;
d) detecting for positive hybridization results; and
e) assessing the mammalian species origin or origins of the sample according to positive hybridization results;
wherein the mammalian species to be identified is/are *Sus scrofa, Bos taurus, Ovis aries, Equus caballus, Canis lupus, Felis catus and Mus musculus.* Further disclosed herein is a method of rapid identification of a mammalian species origin or mammalian species origins of a sample, comprising the steps of:
a) gathering a sample to be tested;
b) processing the sample for use for identification purpose;
c) dividing the sample into a number of portions for situation in a multi-well container or containers;
d) providing a plurality of DNA probesof SEQ ID NOs. 1-241 as shown in below Appendix and the accompanied Sequence Listing or at least 98% sequence identify of the DNA sequences thereof, wherein the number of the sample portions is greater than the number of mammalian species types from which the DNA probes derives;
e) selecting some or all of the DNA probes for hybridization with the sample portions;
f) subjecting the sample portions for intended hybridization with the selected DNA probes simultaneously;
g) detecting for positive hybridization results from the intended hybridization of the sample portions and the selected DNA probes; and
h) assessing the mammalian species origin or origins of the sample according to positive hybridization results.

According to a second aspect of the present invention, there is provided a kit for use in identification of mammalian species origin or mammalian species origins of a sample, comprising DNA probes of SEQ ID NOs. 1-241 derived from a number of different mammalian species. So, a kit for use in rapid identification of mammalian species origin or mammalian species origins of a sample, comprising a plurality of DNA probes derived from a number of different mammalian species and of SEQ ID NOs. 1-241 is provided. In an embodiment, the DNA probes may be immobilized on a suitable substrate, such as on a hybridization membrane or on a wall of a plastic(s) well.

A method of engineering DNA probes for use in rapid identification of mammalian species origin or mammalian species origins of a sample, can comprise the steps of:-
a) identifying regions of DNA sequence with a length from 60 to 80 bases from 48^{th} to 705^{th} bp of the COI gene of a first mammalian species, yielding a first group of DNA regions; and
b) from the first group of DNA regions, identifying regions of DNA sequence meeting the following criteria:-
   i) with GC content from substantially 50 to 52%;
   ii) with a positive value of delta G, yielding a third group of DNA regions;
   iii) in which the hybridization temperature (Thyb) is from 15-25°C below the melting temperature (Tm); and
   iv) in which the difference between the number of secondary structures (SS) and the value of secondary structure (SS) is between 0 to 4; and
c) engineering DNA probes based on the identified DNA regions from said step b).

The delta G value may be larger than 1. The Tm may be substantially 79-83°C. With 50mmole of sodium chloride and without any organic solvent, the Thyb may be larger than 60°C, NaCl w/o any organic solvent. At least some of the DNA probes may have DNA sequences with SEQ ID NOs. 1-241.

A further method of engineering DNA probes for use in rapid identification of mammalian species origin or mammalian species origins of a sample, can comprise the steps of:-
a) identifying regions of DNA sequence with a length from 60 to 80 bases from 48^{th} to 705^{th} bp of the COI gene of a first mammalian species, yielding a first group of DNA regions;
b) from the first group of DNA regions, selecting regions of DNA sequence with GC content from substantially 50 to 52%, yielding a second group of DNA regions;
c) from the second group of DNA regions, selecting regions of DNA sequence with a positive value of delta G, yielding a third group of DNA regions;
d) from the third group of DNA regions, selecting regions of DNA sequence in which the hybridization temperature (Thyb) is from 15-25°C below the melting temperature (Tm), yielding a fourth group of DNA regions;
e) from the fourth group of DNA regions, selecting regions of DNA sequence in which the difference between the number of secondary structures (SS) and the value of secondary structure (SS) is between 0 to 4; and
f) engineering DNA probes based on the identified DNA regions from said step e).

The delta G value may be larger than 1. The Tm may be substantially 79-83°C. With 50mmole of sodium chloride and without any organic solvent, the Thyb may be larger than 60°C, NaCl w/o any organic solvent. At least some of the DNA probes may have DNA sequences with SEQ ID NOs. 1-241.

### BRIEF DESCRIPTION OF DRAWINGS

Some embodiments of the present invention will now be explained, with reference to the accompanied drawings, in which:-
Figure 1 shows images illustrating specificity examination of species-specific probes of each listed mammal species according to an embodiment of the present invention;
Figure 2 is a key showing the pattern of distribution of species specific probes immobilized on the membranes used in experiments, results of which are demonstrated in Figure 1 and Figures 3a to 3d;
Figures 3a to 3d show the effect of hybridization temperature and/or time for hybridization according to an embodiment of the present invention;

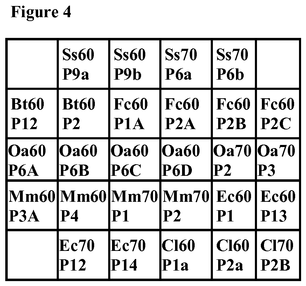

Figure 4 is a key showing the pattern of distribution of species specific probes immobilized on the membrane for multiple species detection in an experiment, results of which are demonstrated in Figure 5; and
Figure 5 shows the effect of multiple species detection according to an embodiment of the present invention.

### DETAILED DESCRIPTION OF PREFERRED EMBODIMENTS OF THE INVENTION

DNA probes or DNA oligo-nucleotides (or oligo-nucleotide sequences) have been widely used for a vast range of applications. The requirements for a DNA probe or a group of DNA probes suitable for use in different applications can be rather different. The usefulness, accuracy and/or efficiency of the applications largely depend on the DNA probe sequence(s). While there has been numerous proposal of making or using DNA probes in different scenario, there is however, no one single principle that can be appropriate for use in all applications. In this context, after much research and development, a number of DNA probes have been engineered, allowing identification of mammalian species including *Bos Taurus* (cattle), *Sus scrofa* (pig), *Ovis aries* (sheep), *Equus caballus* (horse), *Canis lupus* (dog) and *Mus musculus* (mouse) and *Felis Catus* (cat). Accordingly, there is provided a plurality of DNA probes having DNA sequences with SEQ ID NOs. 1-241, as shown in Appendix and the Sequence Listing submitted with this patent specification. It is to be noted that the Appendix shows sequences generally mirroring those in the Sequence Listing although in the Appendix further designations are provided for reference and illustration purposes.) Studies have shown that these particular sequences are sufficiently species specific and are able to detect the relevant species origin of a sample or multiple species origins of a sample, as the case may be. Studies have also shown that sequences having at least 98% sequence identify will likewise produce workable results.

One object of this present invention is to allow rapid identification of a mammalian species origin or a mammalian species origin in one experiment or simultaneously, and efficiently, without having to make use of costly equipment. In the present disclosure, there is provided a method of rapid identification of a mammalian species origin or mammalian species origins of a sample, comprising the steps of a) gathering a sample to be tested, b) processing the sample for use for identification purpose, c) dividing the sample into a number of portions for situation in a multi-well container or containers; d) providing a plurality of DNA probes of SEQ ID NOs. 1-241, wherein the number of the sample portions is greater than the number of mammalian species types from which the DNA probes derives; e) selecting some or all of the DNA probes for hybridization with the sample portions; f) subjecting the sample portions for intended hybridization with the selected DNA probes simultaneously; g) detecting for positive hybridization results from the intended hybridization of the sample portions and the selected DNA probes; and i) assessing the mammalian species origin or origins of the sample according to positive hybridization results.

It is to be noted that with this embodiment, a skilled person would be able to conduct one experiment for detecting the species origin of a sample. Specifically, referring to step f) of subjecting the sample portions for intended hybridization with the selected DNA probes simultaneously, multiple hybridizations can occur simultaneously in one experiment. This translates to efficiency in species origin identification, or rapid identification of species origin(s). If there is only one species origin, e.g. cattle, of the sample, then positive hybridization results will be detected from the hybridization(s) in which the DNA probe(s) is(are) designed from the DNA sequence of that particular species, i.e. cattle. On the other hand, if there are two species origins, e.g. cattle and pig, then positive hybridization results will be detected from the hybridizations in which the DNA probes are designed from the DNA sequences of both the species of cattle and pig. This provides a further technical advantage of allowing rapid identification of multiple species origins in one step. In other words, the present invention allows identification of mammalian species in a mixed population of samples within a single experiment.

As explained above, the usefulness of DNA probes will largely depend on the particular sequence thereof. A method of engineering DNA probes for use in identification in the context of rapid identification of a mammalian species origin or mammalian species origins as explained above is described. The more detailed procedures for designing these DNA probes will then follow.

The DNA sequences used to define those probes are required to possess sufficient differences to distinguish between different species. The COI gene of a number of mammalian species can be utilized. Specifically, studies have shown that the 48^{th} to 705^{th} bp of this gene can be used as the DNA barcode for species identification in animals. The region can be amplified by PCR with the use of universal primers cocktail, making it available to generate a large amount of amplicons for hybridization for identification. Use of this region is made.

Apart from the region of DNA sequence, to design species-specific DNA probes which are able to be used in a single experiment for identifying multiple species, those probes should have highly similar physical and chemical properties for hybridizing at the same environmental conditions. Moreover, other criteria are required to maximize the specificity of those probes. Therefore, criteria concerned for defining those probes include the length, guanine-cytosine (GC) content, delta G, melting temperature and hybridization temperature, and presence of secondary structure.

There is however priority in these criteria when defining those probes. Length generally is the first concerned criterion. In theory, and typically, longer probe will give higher specificity and more efficient during hybridization although longer and yet reliable probe would be more difficult to generate. For example, synthesizing longer oligonucleotides would require enzymatic ligation approach which is more complicated and costly, rendering synthesis of longer probes commercially not possible in companies with less financial resources. Setting aside cost, cross hybridization is also related to the length of the oligonucleotide and is not desirable. Shorter probe will be easier to generate although their specificity will be weaker.

Of course the length of DNA probes depend on the particular type of application, in the context of this invention, studies in the present context have identified that a length from 50-80 bp will be able to achieve the identification satisfactorily. Studies have shown that DNA probes with this length provide the ability to resolve species satisfactorily. However, the length of the DNA probes is from 60-80bp according to the present invention. Based on the criteria discussed above, studies have shown that the present invention have defined probes within conventional DNA barcode region with length at 60, 70 and 80 bp for the mammalian species in Table 1.

The next criterion is GC content, i.e. the percentage of nitrogenous bases, guanine and cytosine, in the target sequence. The requirement on GC content differs, depending on the particular application. There are four different nitrogenous bases in DNA, including guanine (G), cytosine (C), adenine (A) and thymine (T). This might be due to the fact that G and C bind each other by forming 3 hydrogen bonds while A and T form 2 hydrogen bonds, making a DNA sequence with higher GC content more stable, or there could be other reasons. As a result, a higher GC content requires higher melting temperature (Tm) to provide enough energy for denaturing the DNA sequence. Moreover, higher hybridization temperature (T_{hyb}) for the DNA sequences is also resulted. While it has been suggested that the higher GC content increases the specificity in hybridization between oligonucleotide probe and complementary DNA, it does not mean the highest GC content of DNA probes will be suitable in this context. If the GC content is too high, the DNA sequence could become undesirably coiled. Instead, studies have shown that probes with GC content between 50% and 52% is appropriate for accuracy for the above listed mammalian species.

The third criterion is delta G which is the change in Gibbs Free Energy (in units of kcal/mole) between a system and the environment. In DNA or oligonucleotide sequence, when there are two complementary strands presence, they have tendency to form a duplex structure. A positive delta G at a given temperature indicates that the two strands tend to keep in single-stranded status while negative delta G indicates that the two stands tend to bind together to form a double-stranded structure. The DNA probes defined prefer to have positive delta G. The delta G used to define the probes at the hybridization temperature need larger than 1. Therefore, formation of hairpin structures due to self-binding of short complementary sequences within the probe sequence could be minimized.

Melting temperature (Tm) and hybridization temperature (T_{hyb}) are the followed criteria. These two criteria are inter-related. It has been shown that at Tm, 50% of DNA exists in single-stranded status, and there is mathematical equation or simulation for Tm. Generally, the equation derived by thermodynamic basis sets for nearest neighbor interactions is commonly used. On the other hand, hybridization conditions are selected to favor the formation of DNA-probe duplex. Usually, the hybridization temperature (T_{hyb}) is as much as 25°C below the Tm, hence allowing the defined probes to bind to their complementary region on the amplicons. Moreover, the Tm is greatly affected by the GC content, length of the sequence and chemistry conditions, such as salt concentration and presence of organic solvents. Accordingly, the hybridization temperature (Thyb) substantially 25°C below the melting temperature (Tm) is appropriate in allowing and enhancing the hybridization, and thus identification. (However, studies have shown that the hybridization temperature (T_{hyb}) may be from 15-25°C below the Tm.) The melting temperature for the probes with GC content at 50% to 52% is between 79°C to 83°C. Together with the criterion of delta G, the calculated T_{hyb} of the defined probes require above 60°C with 50mmole of sodium chloride and without any organic solvent.

Another criterion concerned is the presence of secondary structure (SS). In single-stranded DNA, SS referred to the structures, hairpins as mentioned in the previous section, formed by self-binding of short complementary sequences within the probe. Secondary structures occurred in oligonucleotide probes will impair the performance of the probes by reducing hybridization efficiency and increasing probe to probe variability. SS of a given single stranded DNA can be calculated and represented in SS value and number of SS. Probe with a SS value close to the number of SS indicates that there is a higher propensity for a nitrogenous base in the probe to be single stranded in all predicted SS. Studies have shown that the difference between the number of secondary structures (SS) and the value of secondary structure (SS) should be between 0 to 4. Preferably, the probes with smallest number of SS and SS value closest to the number of SS are taken into account.

**Table 1: List of targeted species for defining species specific probes in the present invention**

| Common Name | Scientific Name |
|---|---|
| Cattle | Bos taurus |
| Pig | Sus scrofa |
| Sheep | Ovis aries |
| Horse | Equus caballus |
| Dog | Canis lupus |
| Mouse | Mus musculus |

COI gene sequences data, including all full, partial and fragment sequences, of the targeted species can be obtained from GenBank and BOLD databases. When designing the DNA probes, duplicated sequence records between these two databases were removed. After alignment, the 48^{th} to 705^{th}bp fragment from 5' end of each record was extracted. For those species with reference sequence in GenBank database, that particular sequence was used as the template to define the species-specific probes. For those species without reference sequences in GenBank database, the most populated sequence in that species was used as the template to define the probes.

The GC content and Tm of all possible sequences with length of 60, 70 and 80bp from the obtained template sequences were calculated. Those sequences with GC content between 50% and 52% were picked for next step. All delta Gs at 25°C and 65°C of these picked sequences were calculated with the use of online program, ZIPFOLD, DINAMELT server. Delta G value at 25°C and 65°C larger than -4kcal/mol and 1 kcal/mol respectively were selected for further step. At the 3' end, if sequences contained 3 or more contiguous G or C nucleotide within the last 5 nucleotides, those sequences were discarded. Selected sequences were then passed to another online program, DNA-folding form, The Mfold web server for calculating the SS value and predicting the secondary structures at 25°C and 65°C. Those sequences with standard deviation smaller than 1 in the SS value and the number of secondary structure smaller than 3 were selected. If no sequence fulfilled these criteria, those DNA sequences with values closest to these two criteria could be accepted.

After passing the selection with those physical criteria, the specificity of these selected sequences was verified within species and with other species. For intraspecific comparison, the selected sequences were compared with all COI genes of the same species obtained. Any different sequences found were also picked to undergo interspecific comparison. In such situation, a group of probes was defined for identifying that target species. If no difference was found, the sequences can be passed directly to interspecific comparison which was carried out by the BLAST system with GenBank database. In interspecific comparison, results are sorted according to the identity. Sequences that showed any result with identity higher than 97% to other species would not be used as species-specific probes.

Similar procedures described above can be applied for defining species specific probes for other organisms.

In order to appreciate the above novel method for rapid identification of a mammalian species origin or mammalian species origins according to the present invention is to be contrasted with two main conventional approaches, as follows.

### (a) DNA fingerprinting by restriction fragment length polymorphism (RFLP)

This method was first introduced in 1985 for human identification and was subsequently applied to identification of other organisms. PCR amplification of short tandem repeat (STR) and variable number of tandem repeats (VNTR) had developed to improve the method. Endonucleases are used to cut the specific restriction sites on the PCR amplified amplicons, generating a number of small fragments at different sizes. The species specific pattern of different fragments can be observed on agarose gel after electrophoresis or DNA chips. These two detection methods had already applied to identify fish species. The problem is that with this approach, sophisticated procedure or expensive equipment is required, such as southern blotting or DNA chip analyzing machine. Moreover, incomplete digestion may occur and intraspecific variations could alter the number of restriction sites on the sequence. Besides, if the sample contained different species, RFLP cannot distinguish all species within one experiment since the fragment pattern generated is combined by several species specific patterns. Multiple experiment or further analysis to resolve the merged patterns is required.

### (b) DNA sequencing

Due to the accumulation of mutation in the genome, particular genes or DNA regions have enough differences to serve as a marker for species identification. The differences can be notified by sequencing and comparing the target DNA region in different species. With PCR amplification technique, the PCR-sequencing method, which now is recognized as DNA-barcoding, has been applied in species identification for several years. A 658 base pairs (bp) sequence in mitochondrial gene, cytochrome c oxidase I (COI), have examined extensively to identify wild range of animal taxa, including insects, aquatic animals, and birds. U.S. Food and Drug Administration accepted COI-based barcoding as one of the species identification methods for fish. A standard operating procedure (SOP) had been published by FDA after a formal single laboratory validation at Center for Food Safety and Applied Nutrition (CFSAN), FDA). This SOP is intended to replace the LIB No. 4420 now. This conventional DNA barcoding method requires analyzing the sequence of whole barcode. Its application is limited by high operational cost due to the requirement of equipment and time for data analysis. Furthermore, additional costs and steps are required after PCR amplification if samples containing multiple species are examining. PCR amplified amplicons with different species cannot be sequenced in a single experiment. Amplicon separation of different species by bacterial cloning is required. Processes involve ligation, transformation, culture of bacterial colonies on agar plate. Extra step, including picking bacterial colonies, culture of bacterial in broth or even prepping the amplicon ligated plasmids is required if the biotechnology company have not provide those services. It will further increase 2 more days in overall process.

In view of the conventional approaches, and to decrease the cost and shorten the time for species identification, and to further allow the identification of multiple species within one experiment, new identification approach is required. The present invention, as illustrated above, defines species specific DNA sequences served as DNA probes for identifying multiple species in the same experiment through DNA hybridization. The target species involved in the present invention are mammalian species, including *Bos taurus, Sus scrofa, Ovis aries, Equus caballus, Canis lupus* and *Mus musculus.*

It is to be noted that more than one probe may be needed to ascertain whether a sample originates from a particular inter-species or not. This is because in certain region of the COI gene of a particular inter-species, the sequence thereof may differ among intra-species of that particular inter-species. To assess whether the sample originates from the inter-species or not, multiple probes derived from different intra-sequences of all of the known intra-species would be needed. For example, probes with SEQ ID NOs. 7-11 shown in Appendix are all derived from different intra-species of the same inter-species. Accordingly, it is envisaged that the present invention would be capable of detecting not only the inter-species origin(s) of a sample, but also the intra-species origin(s) of a sample.

In order to more clearly illustrate the present invention, the following, with reference to the figures, will demonstrate the elements of the present invention by way of example.

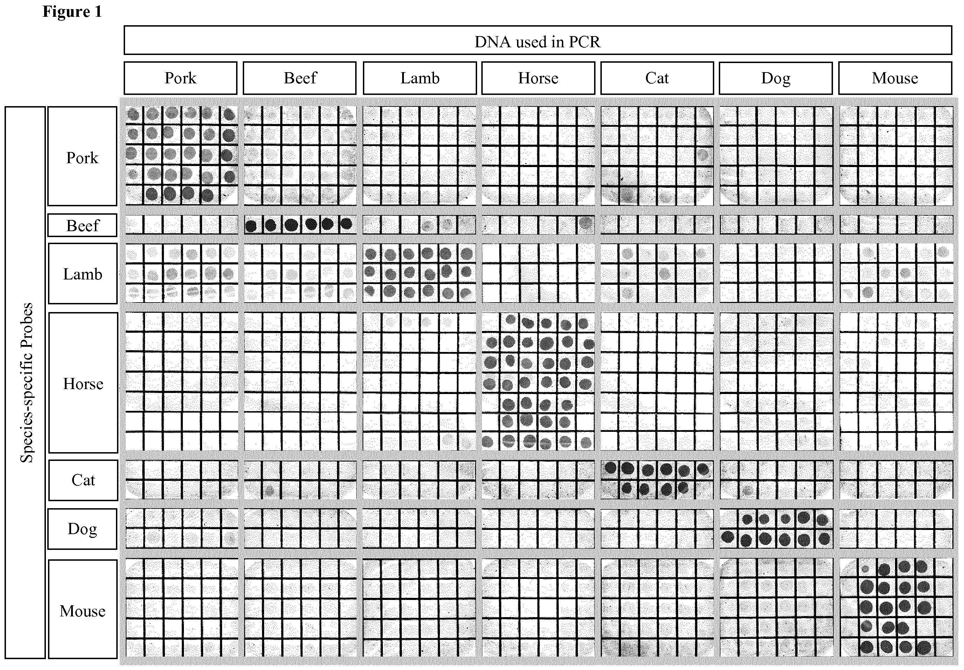

A first experiment was conduct to test whether the engineered DNA probes were able to identify mammalian species samples with sufficient species specificity. In this experiment, the DNA used in each PCR is isolated or originates from a single species. PCR amplicons of each species were hybridized to all probes as indicated in Figure 1. Hybridization temperature was 42°C. 10 min color development for signal detection was applied. Signal was detected after hybridization between species-specific probes.

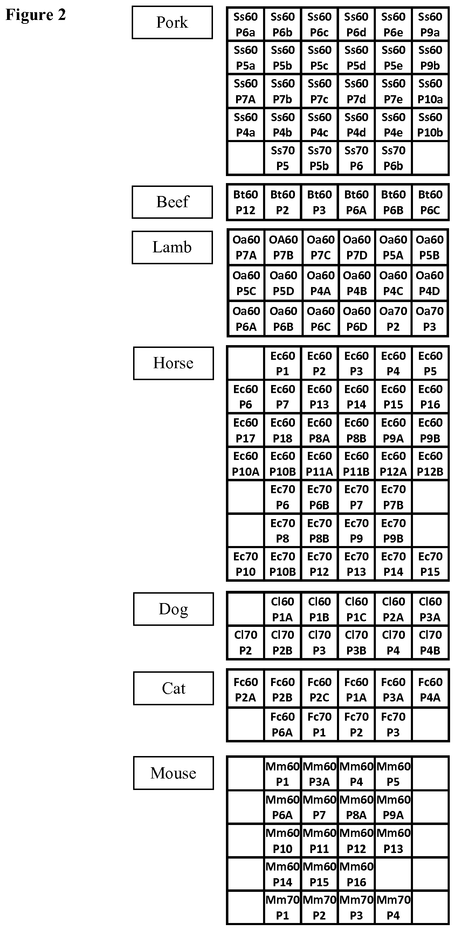

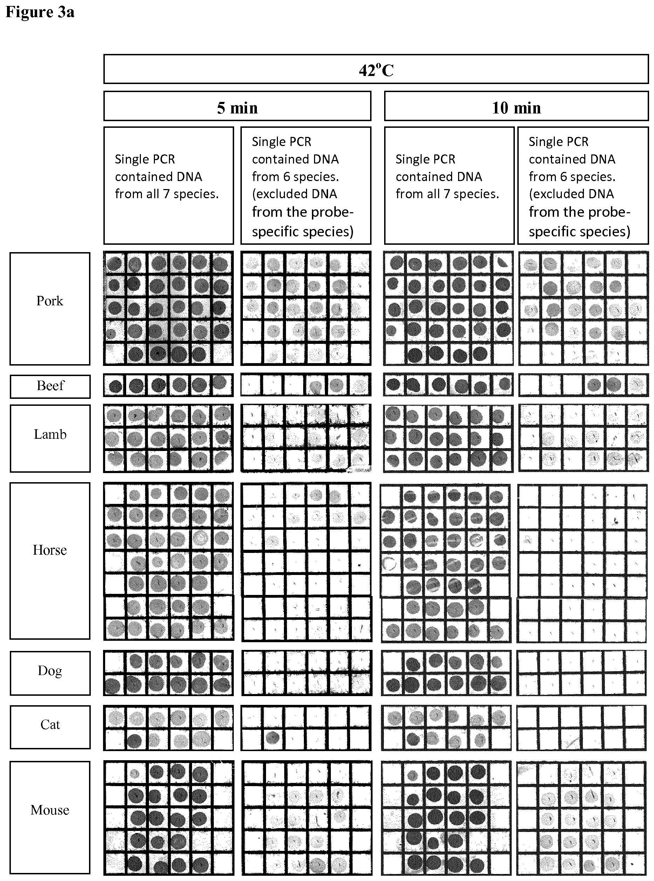

A number of observations can be seen from Figure 1. Please however also refer to Figure 2, Figure 6 and the Sequence Listing. Specifically, Figure 2 is a key showing which probes are located in which wells on the templates. The probes were immobilized on the membranes used in the experiments, results of which are shown in Figures 3a to 3d. The following is also to be noted.
Ss: *Sus scrofa* (Pork)
Bt: *Bos taurus* (Beef)
Oa: *Ovis aries* (Lamb)
Ec: *Equus caballus* (Horse)
CI: *Canis lupus* (Dog)
Fc: *Felis catus* (Cat)
Mm: *Mus musculus* (Mouse)

For beef, probe P6 showed weak non-specific binding with *Ovis aries* (lamb) and *Equus caballus* (horse).

For pork, weak non-specific binding signal to *Bos taurus* (beef) was found in some probes. Non-specific binding signal to *Felis catus* (cat) were also found in probe P10a (60bp), and probe P5a and P6a (70bp).

For horse, Probes P1 to P4 showed weak non-specific signal against *Ovis aries* (lamb) and *Canis lupus* (dog).

70bp Weak non-specific binding signal were observed in P14 and P15 against *Canis lupus.*

For cat, only probe P6 with 60 bp in length showed non-specific binding with *Bos taurus.*

For dog, All probes are specific to *Canis lupus.*

For mouse, All probes were specific to *Mus musculus* (mouse). Weak non-specific binding signal were found in probes with 70 bp length, P3 and P4, against *Canis lupus.*

For lamb, Probes P4a-4d, P5d, P6a-d and P7d in 60 bp length showed non-specific binding signal to *Sus scrofa* (pork). Probes P4b, P5b, P6b and P7b also showed non-specific binding signal to *Felis catis* and *Mus musculus.*

In another series of experiments, temperature for hybridization and time for hybridization for the identification of the species using the probes were studied.

In Figure 3a, it is shown that the temperature for hybridization was at 42°C. Some non-specific signal was found in pork, beef, horse, cat and mouse specific probes. However, at least 2 to 3 probes in each species were specific enough at these hybridization conditions. Stronger signal was found when color development time was increased from 5 min to 10 min.

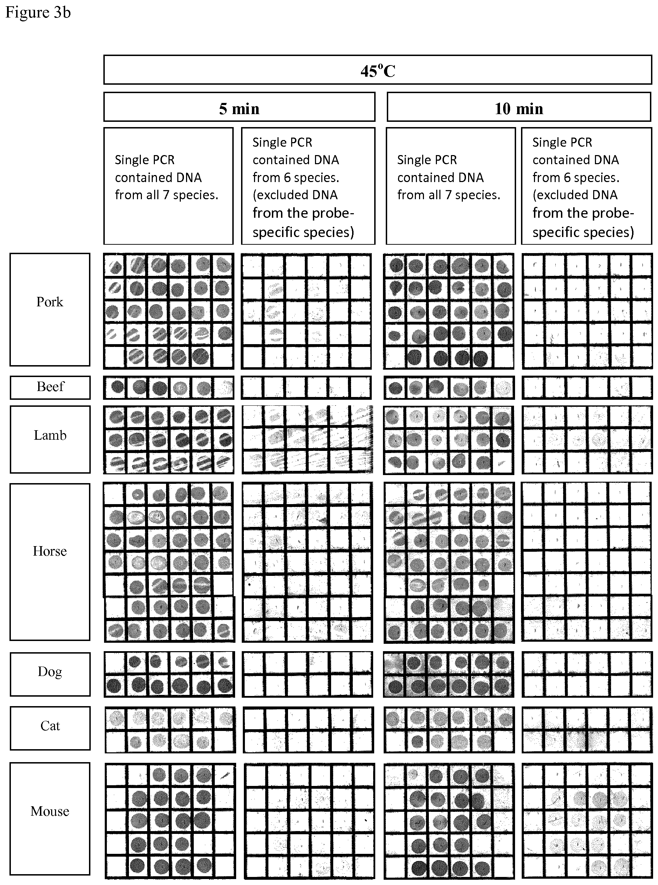

In Figure 3b, the temperature for hybridization was increased from 42°C to 45°C. Signal had become more specific. Nearly all probes were specific at 5 min color development time. Stronger signal were found when color development (hybridization) time was increased from 5 min to 10 min, but some non-specific signals were found in several mouse specific probes.

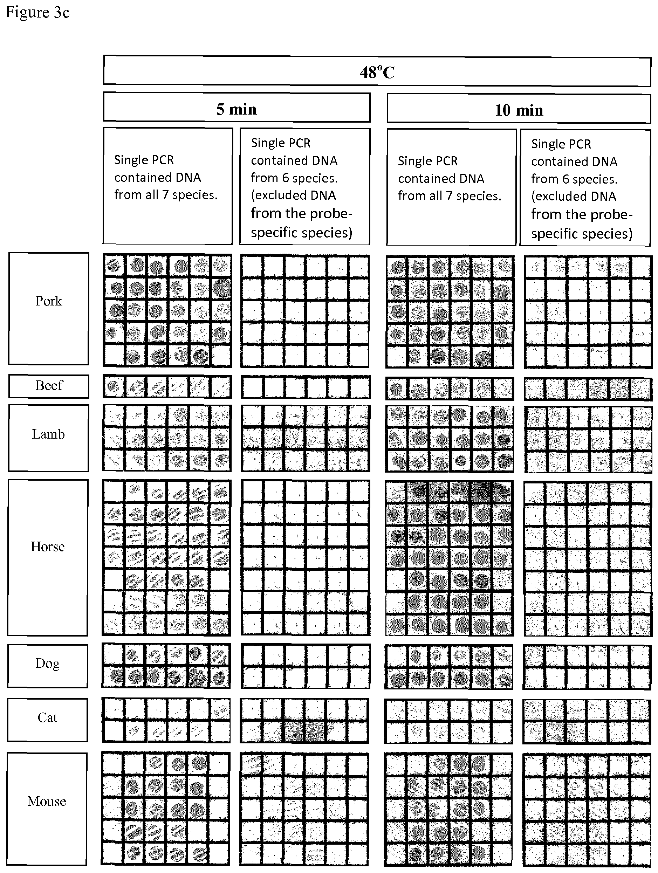

In Figure 3c, signal became weaker when temperature was increased to 48°C. The cat specific probes nearly gave no signal. Apart from the cat specific probes, strong and specific signals were found in other species when color development time was increased to 10 min.

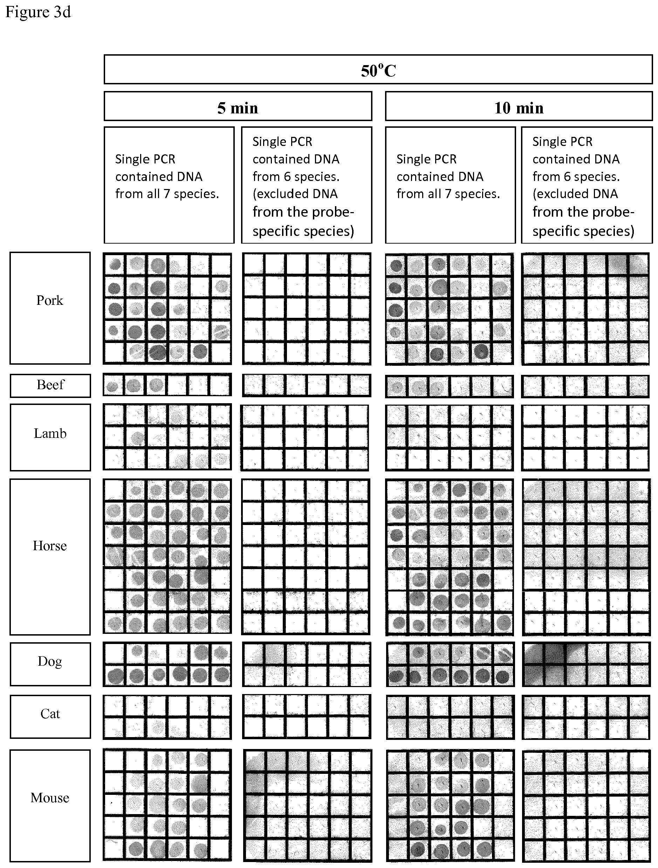

In Figure 3d, signal became weak at 50°C. Nearly no signal could be detected for lamb and cat specific probes. However, most probes for pork, beef, horse, dog and mouse still gave specific signal.

In the experiments as shown in Figures 3a to 3d, it can be seen that the specific engineered probes are specific enough, although the working temperature for each species for optimal identification may vary. From all hybridization conditions, the hybridization temperature of about 45°C yields better specific signal for all species. Several probes can be selected at this condition and place in the same membrane for a multi-species detection. The duration for color development is between about 5 to 10 min.

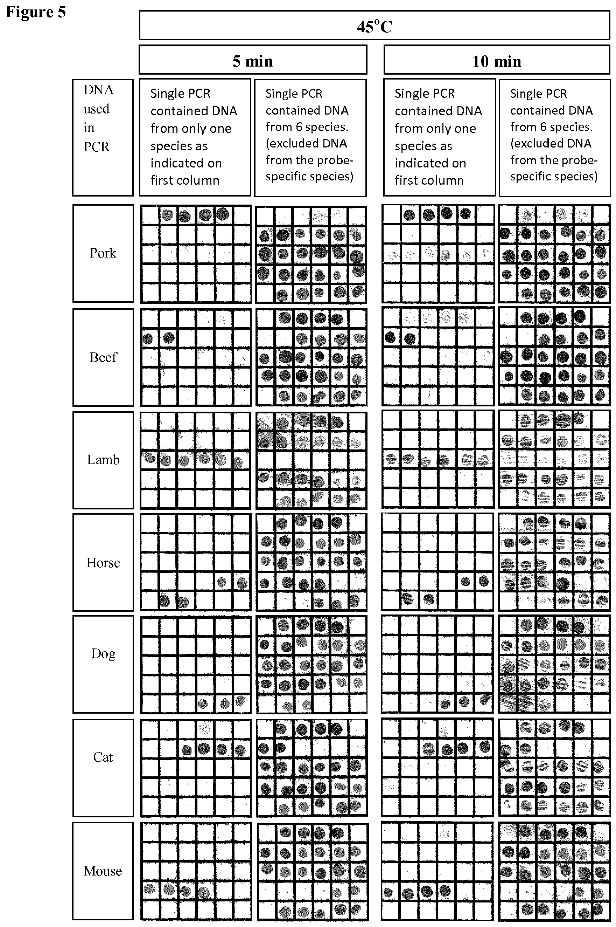

In another experiment, it is shown that the present invention can be applied for multiple species detection simultaneously. Referring to Figure 5, there is shown a panel of species specific probes selected from each mammal species that are immobilized on the same membrane for multiple species detection. Please refer to Figure 2 for the locations of the different probes in the panel.

Specific probes from each species were selected and immobilized on same membrane. The specificity of these probes in mixed samples was examined. For 5 min color development (hybridization time), it is shown that the engineered probes are specific to their own species. No non-specific signals were found when hybridized with PCR product amplified from DNA of other species. For 10 min of color development, signal was stronger but some non-specific signals were found although the identification was still satisfactory.

It is to be noted that the following a skilled person in the art possess the skills disclosed in the following reference.
Gill, P., Jeffreys, A.J. and Werrett, D.J., (1985), Forensic application of DNA 'fingerprints'. Nature, 12-18; 318 (6046): p577-579.
Lockley, A.K. and Bardsley, R.G., (2000), DNA-based methods for food authentication. Trends in Food Science and Technology, 11, p67-77.
Handy, S.M., Deeds, J.R., Ivanova, N.V., Hebert, P.D.N., Hanner, R., Ormos, A., Weigt, L.A., Moore, M.M. and Yancy, H.F. (2011). A single laboratory validated method for the generation of DNA barcodes for the identification of fish for regulatory compliance. Journal of AOAC International. 94 (1), 201-210.
Meusnier, I., Singer, G.A., Landry, J.F., Hickey, D.A., Hebert, P.D. and Hajibabaei, M., (2011), A universal DNA mini-barcode for biodiversity analysis, BMC Genomics, 9, p214.
Shokralla, S., Zhou, X., Janzen, D.H., Hallwachs, W., Landry, J.F., Jacobus, L.M. and Hajibabaei, M., (2011), Pyrosequencing for mini-barcoding of fresh and old museum specimens, PLoS One, 6(7):e21252, Epub 2011 Jul 27.
Chou, C.C., Chen, C.H., Lee, T.T. and Peck, K., (2004), Optimization of probe length and the number of probes per gene for optimal microarray analysis of gene expression, Nucleic Acids Research, 32(12), e99.
Aquino de Muro, M., (2008), Probe Design, Production, and Applications, Molecular Biomethods Handbook, A, p41-53.

## Claims

1. A method of simultaneous identification of different mammalian species origins of a sample, comprising the steps of:
a) dividing the sample into a number of portions and situating the sample portions in a multi-well container or containers for use in DNA hybridization;
b) providing DNA probes of SEQ ID NOs. 1-241 with a length from 60-80 bases, wherein the number of the sample portions is greater than the number of mammalian species from which the DNA probes derive;
c) hybridizing the sample portions with the DNA probes;
d) detecting for positive hybridization results; and
e) assessing the mammalian species origin or origins of the sample according to positive hybridization results;
wherein the mammalian species to be identified is/are *Sus scrofa, Bos taurus, Ovis aries, Equus caballus, Canis lupus, Felis catus and Mus musculus.*

2. A method as claimed in Claim 1, wherein in step c), a temperature for hybridization from 42-48°C is set.

3. A kit for use in identification of mammalian species origin or mammalian species origins of a sample, comprising DNA probes of SEQ ID NOs. 1-241 derived from a number of different mammalian species .

## Patentansprüche

1. Verfahren zur simultanen Identifizierung von verschiedenen Säugetier-Spezies einer Probe, aufweisend die Schritte:
a) Teilen der Probe in eine Anzahl von Portionen und Unterbringen der Probenportionen in einen Multi-Well-Behälter oder Behälter zur Verwendung bei einer DNA-Hybridisierung;
b) Bereitstellen von DNA-Sonden von SEQ ID Nr. 1-241 mit einer Länge von 60-80 Basen, wobei die Anzahl der Probenportionen größer ist als die Anzahl der Säugetier-Spezies, von denen sich die DNA-Sonden ableiten;
c) Hybridisieren der Probenportionen mit den DNA-Sonden;
d) Detektieren auf positive Hybridisierungsresultate; und
e) Festlegen des Ursprungs der Säugetier-Spezies oder der Ursprünge der Probe entsprechend der positiven Hybridisierungsresultate;
wobei die zu identifizierende/n Säugetier-Spezies ist/sind *Sus scrofa, Bos taurus, Ovis aries, Equus caballus, Canis lupus, Felis catus und Mus musculus.*

2. Verfahren nach Anspruch 1, wobei in Schritt c) eine Temperatur zur Hybridisierung auf 42-48°C gesetzt wird.

3. Kit zur Verwendung bei einer Identifizierung eines Säugetier-Spezies-Ursprungs oder von Säugetier-Spezies-Ursprüngen einer Probe, aufweisend DNA-Sonden mit SEQ ID Nr. 1-241, die von einer Anzahl an verschiedenen Säugetier-Spezies abgeleitet sind.

## Revendications

1. Un procédé pour l'identification simultanée d'origines de différentes espèces de mammifères d'un échantillon, comprenant les étapes:
a) diviser l'échantillon dans un nombre de portions et logeant les portions d'échantillon dans un récipient or des récipients à puits multiples pour l'utilisation en hybridation d'ADN;
b) fournir des sondes d'ADN avec SEQ ID NOs. 1-241 ayant une longueur de 60-80 bases, dans lequel le nombre de portions d'échantillon est supérieur au nombre d'espèces de mammifères dont dérivent les sondes d'ADN;
c) hybrider les portions d'échantillon avec les sondes d'ADN;
d) détecter des résultats d'hybridation positive; et
e) évaluer l'origine d'espèces de mammifères ou les origines de l'échantillon en fonction des résultats d'hybridation positifs;
dans lequel l'espèce ou les espèces de mammifères à identifier est/sont *Sus scrofa, Bos taurus, Ovis aries, Equus caballus, Canis lupus, Felis catus et Mus musculus.*

2. Le procédé selon la revendication 1, dans lequel à l'étape c), on règle la température pour l'hybridation de 42 à 48 ° C.

3. Un kit à utiliser pour identifier une origine d'espèces de mammifères ou des origines d'espèces de mammifères d'un échantillon, comprenant des sondes d'ADN avec SEQ ID NOs. 1-241 dérivées d'un nombre d'espèces mammifères différentes.


### SEQUENCE LISTING

<110> CHENG, SHUK HAN LIN, CHUN CHI
<120> METHOD AND MEANS FOR IDENTIFICATION OF ANIMAL SPECIES
<130> 2958.014A
<140>
   <141>
<150> 61/608,824
   <151> 2012-03-09
<160> 241
<170> PatentIn version 3.5
<210> 1
   <211> 60
   <212> DNA
   <213> Artificial Sequence
<220>
   <223> Description of Artificial Sequence: Synthetic probe
<400> 1
   tccgtaataa ttaccgccgt actactacta ctctcgctcc ctgtattagc agccggcatc 60
<210> 2
   <211> 60
   <212> DNA
   <213> Artificial Sequence
<220>
   <223> Description of Artificial Sequence: Synthetic probe
<400> 2
   ccgtaataat taccgccgta ctactactac tctcgctccc tgtattagca gccggcatca 60
<210> 3
   <211> 60
   <212> DNA
   <213> Artificial Sequence
<220>
   <223> Description of Artificial Sequence: Synthetic probe
<400> 3
   cgtaataatt accgccgtac tactactact ctcgctccct gtattagcag ccggcatcac 60
<210> 4
   <211> 60
   <212> DNA
   <213> Artificial Sequence
<220>
   <223> Description of Artificial Sequence: Synthetic probe
<400> 4
   ccggaaccta aatacaacct tcttcgaccc ggcaggagga ggagacccta ttctatatca 60
<210> 5
   <211> 60
   <212> DNA
   <213> Artificial Sequence
<220>
   <223> Description of Artificial Sequence: Synthetic probe
<400> 5
   ccgaaaccta aatacaacct tcttcgaccc ggcaggagga ggagacccta ttctatatca 60
<210> 6
   <211> 60
   <212> DNA
   <213> Artificial Sequence
<220>
   <223> Description of Artificial Sequence: Synthetic probe
<400> 6
   ccggaaccta aatacaactt tcttcgaccc ggcaggagga ggagatccta ttctatacca 60
<210> 7
   <211> 60
   <212> DNA
   <213> Artificial Sequence
<220>
   <223> Description of Artificial Sequence: Synthetic probe
<400> 7
   agcctactaa ttcgcgctga actaggtcag cccggaaccc tacttggcga tgatcaaatc 60
<210> 8
   <211> 60
   <212> DNA
   <213> Artificial Sequence
<220>
   <223> Description of Artificial Sequence: Synthetic probe
<400> 8
   agcctactaa ttcgcgctga actaggtcag cccggaactc tacttggcga tgatcaaatc 60
<210> 9
   <211> 60
   <212> DNA
   <213> Artificial Sequence
<220>
   <223> Description of Artificial Sequence: Synthetic probe
<400> 9
   agcctgctaa ttcgcgctga actaggtcag cccggaaccc tacttggcga tgatcaaatc 60
<210> 10
   <211> 60
   <212> DNA
   <213> Artificial Sequence
<220>
   <223> Description of Artificial Sequence: Synthetic probe
<400> 10
   agcctactaa ttcgcgctga actaggtcag cccggaaccc tacttcccca tgatcaaatc 60
<210> 11
   <211> 60
   <212> DNA
   <213> Artificial Sequence
<220>
   <223> Description of Artificial Sequence: Synthetic probe
<400> 11
   agcctactaa ttcgcgctga actaggtcag cccggaactt tacttggcga tgaccaaatc 60
<210> 12
   <211> 60
   <212> DNA
   <213> Artificial Sequence
<220>
   <223> Description of Artificial Sequence: Synthetic probe
<400> 12
   ttgagcctac taattcgcgc tgaactaggt cagcccggaa ccctacttgg cgatgatcaa 60
<210> 13
   <211> 60
   <212> DNA
   <213> Artificial Sequence
<220>
   <223> Description of Artificial Sequence: Synthetic probe
<400> 13
   ttgagcctac taattcgcgc tgaactaggt cagcccggaa ctctacttgg cgatgatcaa 60
<210> 14
   <211> 60
   <212> DNA
   <213> Artificial Sequence
<220>
   <223> Description of Artificial Sequence: Synthetic probe
<400> 14
   ttgagcctgc taattcgcgc tgaactaggt cagcccggaa ccctacttgg cgatgatcaa 60
<210> 15
   <211> 60
   <212> DNA
   <213> Artificial Sequence
<220>
   <223> Description of Artificial Sequence: Synthetic probe
<400> 15
   ttgagcctac taattcgcgc tgaactaggt cagcccggaa ccctacttcc ccatgatcaa 60
<210> 16
   <211> 60
   <212> DNA
   <213> Artificial Sequence
<220>
   <223> Description of Artificial Sequence: Synthetic probe
<400> 16
   ttaagcctac taattcgcgc tgaactaggt cagcccggaa ctttacttgg cgatgaccaa 60
<210> 17
   <211> 60
   <212> DNA
   <213> Artificial Sequence
<220>
   <223> Description of Artificial Sequence: Synthetic probe
<400> 17
   tgagcctact aattcgcgct gaactaggtc agcccggaac cctacttggc gatgatcaaa 60
<210> 18
   <211> 60
   <212> DNA
   <213> Artificial Sequence
<220>
   <223> Description of Artificial Sequence: Synthetic probe
<400> 18
   tgagcctact aattcgcgct gaactaggtc agcccggaac tctacttggc gatgatcaaa 60
<210> 19
   <211> 60
   <212> DNA
   <213> Artificial Sequence
<220>
   <223> Description of Artificial Sequence: Synthetic probe
<400> 19
   tgagcctgct aattcgcgct gaactaggtc agcccggaac cctacttggc gatgatcaaa 60
<210> 20
   <211> 60
   <212> DNA
   <213> Artificial Sequence
<220>
   <223> Description of Artificial Sequence: Synthetic probe
<400> 20
   tgagcctact aattcgcgct gaactaggtc agcccggaac cctacttccc catgatcaaa 60
<210> 21
   <211> 60
   <212> DNA
   <213> Artificial Sequence
<220>
   <223> Description of Artificial Sequence: Synthetic probe
<400> 21
   taagcctact aattcgcgct gaactaggtc agcccggaac tttacttggc gatgaccaaa 60
<210> 22
   <211> 60
   <212> DNA
   <213> Artificial Sequence
<220>
   <223> Description of Artificial Sequence: Synthetic probe
<400> 22
   gagcctacta attcgcgctg aactaggtca gcccggaacc ctacttggcg atgatcaaat 60
<210> 23
   <211> 60
   <212> DNA
   <213> Artificial Sequence
<220>
   <223> Description of Artificial Sequence: Synthetic probe
<400> 23
   gagcctacta attcgcgctg aactaggtca gcccggaact ctacttggcg atgatcaaat 60
<210> 24
   <211> 60
   <212> DNA
   <213> Artificial Sequence
<220>
   <223> Description of Artificial Sequence: Synthetic probe
<400> 24
   gagcctgcta attcgcgctg aactaggtca gcccggaacc ctacttggcg atgatcaaat 60
<210> 25
   <211> 60
   <212> DNA
   <213> Artificial Sequence
<220>
   <223> Description of Artificial Sequence: Synthetic probe
<400> 25
   gagcctacta attcgcgctg aactaggtca gcccggaacc ctacttcccc atgatcaaat 60
<210> 26
   <211> 60
   <212> DNA
   <213> Artificial Sequence
<220>
   <223> Description of Artificial Sequence: Synthetic probe
<400> 26
   aagcctacta attcgcgctg aactaggtca gcccggaact ttacttggcg atgaccaaat 60
<210> 27
   <211> 60
   <212> DNA
   <213> Artificial Sequence
<220>
   <223> Description of Artificial Sequence: Synthetic probe
<400> 27
   aagccggagc gggtactgga tgaactgtat acccaccttt agctggaaac ttagcccatg 60
<210> 28
   <211> 60
   <212> DNA
   <213> Artificial Sequence
<220>
   <223> Description of Artificial Sequence: Synthetic probe
<400> 28
   aagccggggc gggtactgga tgaaccgtat acccaccttt agctggaaac ttagcccatg 60
<210> 29
   <211> 60
   <212> DNA
   <213> Artificial Sequence
<220>
   <223> Description of Artificial Sequence: Synthetic probe
<400> 29
   aagccggggc gggtactgga tgaaccgtat acccaccttt agccggaaac ttagcccatg 60
<210> 30
   <211> 60
   <212> DNA
   <213> Artificial Sequence
<220>
   <223> Description of Artificial Sequence: Synthetic probe
<400> 30
   aggccggagc gggtactgga tgaactgtat acccgccttt agctggaaac ttagcccacg 60
<210> 31
   <211> 60
   <212> DNA
   <213> Artificial Sequence
<220>
   <223> Description of Artificial Sequence: Synthetic probe
<400> 31
   aggccggagc gggtactgga tgaactgtat acccaccttt agctggaaac ttagcccacg 60
<210> 32
   <211> 60
   <212> DNA
   <213> Artificial Sequence
<220>
   <223> Description of Artificial Sequence: Synthetic probe
<400> 32
   aagccggagc gggtactgga tgaaccgtat acccaccttt agctggaaac ttagcccatg 60
<210> 33
   <211> 60
   <212> DNA
   <213> Artificial Sequence
<220>
   <223> Description of Artificial Sequence: Synthetic probe
<400> 33
   aagccggggc gggcactgga tgaaccgtat acccaccttt agctggaaac ttagcccatg 60
<210> 34
   <211> 60
   <212> DNA
   <213> Artificial Sequence
<220>
   <223> Description of Artificial Sequence: Synthetic probe
<400> 34
   aagccggggc gggtaccgga tgaaccgtat acccaccttt agctggaaac ttagcccatg 60
<210> 35
   <211> 60
   <212> DNA
   <213> Artificial Sequence
<220>
   <223> Description of Artificial Sequence: Synthetic probe
<400> 35
   aagccggggc gggtactgga tgagccgtat acccaccttt agctggaaac ttagcccatg 60
<210> 36
   <211> 60
   <212> DNA
   <213> Artificial Sequence
<220>
   <223> Description of Artificial Sequence: Synthetic probe
<400> 36
   aagccggagc gggtactgga tgaaccgtat acccaccttt agctggaaac ttagcccatg 60
<210> 37
   <211> 60
   <212> DNA
   <213> Artificial Sequence
<220>
   <223> Description of Artificial Sequence: Synthetic probe
<400> 37
   aagccggggc gggtactgga tgaaccgtat acccaccttt agctggaaac ttagcccgtg 60
<210> 38
   <211> 60
   <212> DNA
   <213> Artificial Sequence
<220>
   <223> Description of Artificial Sequence: Synthetic probe
<400> 38
   aagccggagc gggtactgga tgaactgtat atccaccttt agctggaaac ttggcccatg 60
<210> 39
   <211> 60
   <212> DNA
   <213> Artificial Sequence
<220>
   <223> Description of Artificial Sequence: Synthetic probe
<400> 39
   aagccggggc gggtactgga tgaaccgaat acccaccctt agctggaaac ttagcccaag 60
<210> 40
   <211> 60
   <212> DNA
   <213> Artificial Sequence
<220>
   <223> Description of Artificial Sequence: Synthetic probe
<400> 40
   aagccggggc gggtactgga tgaaccgtat acccaccttt agctggaaac ttaccccatg 60
<210> 41
   <211> 60
   <212> DNA
   <213> Artificial Sequence
<220>
   <223> Description of Artificial Sequence: Synthetic probe
<400> 41
   ggcatcctca atagtagaag ccggagcggg tactggatga actgtatacc cacctttagc 60
<210> 42
   <211> 60
   <212> DNA
   <213> Artificial Sequence
<220>
   <223> Description of Artificial Sequence: Synthetic probe
<400> 42
   ggcatcctca atagtagaag ccggggcggg tactggatga accgtatacc cacctttagc 60
<210> 43
   <211> 60
   <212> DNA
   <213> Artificial Sequence
<220>
   <223> Description of Artificial Sequence: Synthetic probe
<400> 43
   ggcatcctca atagtagaag ccggggcggg cactggatga accgtatacc cacctttagc 60
<210> 44
   <211> 60
   <212> DNA
   <213> Artificial Sequence
<220>
   <223> Description of Artificial Sequence: Synthetic probe
<400> 44
   ggcatcctca atagtagaag ccggggcggg tactggatga accgaatacc cacccttagc 60
<210> 45
   <211> 60
   <212> DNA
   <213> Artificial Sequence
<220>
   <223> Description of Artificial Sequence: Synthetic probe
<400> 45
   ggcatcctca atagtaaaag ccggggcggg tactggatga accgtatacc cacctttagc 60
<210> 46
   <211> 60
   <212> DNA
   <213> Artificial Sequence
<220>
   <223> Description of Artificial Sequence: Synthetic probe
<400> 46
   ggcatcctca atagtagaag ccggggcggg taccggatga accgtatacc cacctttagc 60
<210> 47
   <211> 60
   <212> DNA
   <213> Artificial Sequence
<220>
   <223> Description of Artificial Sequence: Synthetic probe
<400> 47
   ggcatcctca atagtagaag ccggagcggg tactggatga accgtatacc cacctttagc 60
<210> 48
   <211> 60
   <212> DNA
   <213> Artificial Sequence
<220>
   <223> Description of Artificial Sequence: Synthetic probe
<400> 48
   ggcatcctca atagtagaag ccggggcggg tactggatga gccgtatacc cacctttagc 60
<210> 49
   <211> 60
   <212> DNA
   <213> Artificial Sequence
<220>
   <223> Description of Artificial Sequence: Synthetic probe
<400> 49
   ggcatcctca atagtagaag ccggagcggg tactggatga actgtatatc cacctttagc 60
<210> 50
   <211> 60
   <212> DNA
   <213> Artificial Sequence
<220>
   <223> Description of Artificial Sequence: Synthetic probe
<400> 50
   ggcatcctca atagtagagg ccggagcggg tactggatga actgtatacc cgcctttagc 60
<210> 51
   <211> 60
   <212> DNA
   <213> Artificial Sequence
<220>
   <223> Description of Artificial Sequence: Synthetic probe
<400> 51
   ggcatcctca atagtagagg ccggagcggg tactggatga actgtatacc cacctttagc 60
<210> 52
   <211> 60
   <212> DNA
   <213> Artificial Sequence
<220>
   <223> Description of Artificial Sequence: Synthetic probe
<400> 52
   ggcgtcctca atagtagaag ccggagcggg tactggatga actgtatacc cacctttagc 60
<210> 53
   <211> 70
   <212> DNA
   <213> Artificial Sequence
<220>
   <223> Description of Artificial Sequence: Synthetic probe
<400> 53
   caatagtaga agccggagcg ggtactggat gaactgtata cccaccttta gctggaaact 60
   tagcccatgc 70
<210> 54
   <211> 70
   <212> DNA
   <213> Artificial Sequence
<220>
   <223> Description of Artificial Sequence: Synthetic probe
<400> 54
   caatagtaga agccggggcg ggtactggat gaaccgtata cccaccttta gctggaaact 60
   tagcccatgc 70
<210> 55
   <211> 70
   <212> DNA
   <213> Artificial Sequence
<220>
   <223> Description of Artificial Sequence: Synthetic probe
<400> 55
   caatagtaga agccggagcg ggtactggat gaaccgtata cccaccttta gctggaaact 60
   tagcccatgc 70
<210> 56
   <211> 70
   <212> DNA
   <213> Artificial Sequence
<220>
   <223> Description of Artificial Sequence: Synthetic probe
<400> 56
   caatagtaga ggccggagcg ggtactggat gaactgtata cccaccttta gctggaaact 60
   tagcccacgc 70
<210> 57
   <211> 70
   <212> DNA
   <213> Artificial Sequence
<220>
   <223> Description of Artificial Sequence: Synthetic probe
<400> 57
   caatagtaga ggccggagcg ggtactggat gaactgtata cccgccttta gctggaaact 60
   tagcccacgc 70
<210> 58
   <211> 70
   <212> DNA
   <213> Artificial Sequence
<220>
   <223> Description of Artificial Sequence: Synthetic probe
<400> 58
   caatagtaga agccggggcg ggtactggat gagccgtata cccaccttta gctggaaact 60
   tagcccatgc 70
<210> 59
   <211> 70
   <212> DNA
   <213> Artificial Sequence
<220>
   <223> Description of Artificial Sequence: Synthetic probe
<400> 59
   caatagtaga agccggggcg ggcactggat gaaccgtata cccaccttta gctggaaact 60
   tagcccatgc 70
<210> 60
   <211> 70
   <212> DNA
   <213> Artificial Sequence
<220>
   <223> Description of Artificial Sequence: Synthetic probe
<400> 60
   caatagtaga agccggggcg ggtactggat gaaccgtata cccaccttta gctggaaact 60
   taccccatgc 70
<210> 61
   <211> 70
   <212> DNA
   <213> Artificial Sequence
<220>
   <223> Description of Artificial Sequence: Synthetic probe
<400> 61
   caatagtaga agccggggcg ggtactggat gaaccgaata cccaccctta gctggaaact 60
   tagcccaagc 70
<210> 62
   <211> 70
   <212> DNA
   <213> Artificial Sequence
<220>
   <223> Description of Artificial Sequence: Synthetic probe
<400> 62
   caatagtaga agccggggcg ggtactggat gaaccgtata cccaccttta gctggaaact 60
   tagcccgtgc 70
<210> 63
   <211> 70
   <212> DNA
   <213> Artificial Sequence
<220>
   <223> Description of Artificial Sequence: Synthetic probe
<400> 63
   caatagtaga agccggggcg ggtactggat gaaccgtata cccaccttta gccggaaact 60
   tagcccatgc 70
<210> 64
   <211> 70
   <212> DNA
   <213> Artificial Sequence
<220>
   <223> Description of Artificial Sequence: Synthetic probe
<400> 64
   caatagtaga agccggggcg ggtaccggat gaaccgtata cccaccttta gctggaaact    60
   tagcccatgc                                                            70
<210> 65
   <211> 70
   <212> DNA
   <213> Artificial Sequence
<220>
   <223> Description of Artificial Sequence: Synthetic probe
<400> 65
   caatagtaaa agccggggcg ggtactggat gaaccgtata cccaccttta gctggaaact    60
   tagcccatgc                                                            70
<210> 66
   <211> 70
   <212> DNA
   <213> Artificial Sequence
<220>
   <223> Description of Artificial Sequence: Synthetic probe
<400> 66
   caatagtaga agccggagcg ggtactggat gaactgtata tccaccttta gctggaaact    60
   tggcccatgc                                                            70
<210> 67
   <211> 70
   <212> DNA
   <213> Artificial Sequence
<220>
   <223> Description of Artificial Sequence: Synthetic probe
<400> 67
   atagtagaag ccggagcggg tactggatga actgtatacc cacctttagc tggaaactta    60
   gcccatgcag                                                            70
<210> 68
   <211> 70
   <212> DNA
   <213> Artificial Sequence
<220>
   <223> Description of Artificial Sequence: Synthetic probe
<400> 68
   atagtagaag ccggggcggg tactggatga accgtatacc cacctttagc tggaaactta 60
   gcccatgcag 70
<210> 69
   <211> 70
   <212> DNA
   <213> Artificial Sequence
<220>
   <223> Description of Artificial Sequence: Synthetic probe
<400> 69
   atagtagagg ccggagcggg tactggatga actgtatacc cacctttagc tggaaactta 60
   gcccacgcag 70
<210> 70
   <211> 70
   <212> DNA
   <213> Artificial Sequence
<220>
   <223> Description of Artificial Sequence: Synthetic probe
<400> 70
   atagtagagg ccggagcggg tactggatga actgtatacc cgcctttagc tggaaactta 60
   gcccacgcag 70
<210> 71
   <211> 70
   <212> DNA
   <213> Artificial Sequence
<220>
   <223> Description of Artificial Sequence: Synthetic probe
<400> 71
   atagtagaag ccggggcggg tactggatga gccgtatacc cacctttagc tggaaactta 60
   gcccatgcag 70
<210> 72
   <211> 70
   <212> DNA
   <213> Artificial Sequence
<220>
   <223> Description of Artificial Sequence: Synthetic probe
<400> 72
   atagtagaag ccggggcggg cactggatga accgtatacc cacctttagc tggaaactta 60
   gcccatgcag 70
<210> 73
   <211> 70
   <212> DNA
   <213> Artificial Sequence
<220>
   <223> Description of Artificial Sequence: Synthetic probe
<400> 73
   atagtagaag ccggggcggg tactggatga accgtatacc cacctttagc tggaaactta 60
   ccccatgcag 70
<210> 74
   <211> 70
   <212> DNA
   <213> Artificial Sequence
<220>
   <223> Description of Artificial Sequence: Synthetic probe
<400> 74
   atagtagaag ccggggcggg tactggatga accgaatacc cacccttagc tggaaactta 60
   gcccaagcag 70
<210> 75
   <211> 70
   <212> DNA
   <213> Artificial Sequence
<220>
   <223> Description of Artificial Sequence: Synthetic probe
<400> 75
   atagtagaag ccggggcggg tactggatga accgtatacc cacctttagc tggaaactta 60
   gcccgtgcag 70
<210> 76
   <211> 70
   <212> DNA
   <213> Artificial Sequence
<220>
   <223> Description of Artificial Sequence: Synthetic probe
<400> 76
   atagtagaag ccggggcggg tactggatga accgtatacc cacctttagc cggaaactta 60
   gcccatgcag 70
<210> 77
   <211> 70
   <212> DNA
   <213> Artificial Sequence
<220>
   <223> Description of Artificial Sequence: Synthetic probe
<400> 77
   atagtagaag ccggggcggg taccggatga accgtatacc cacctttagc tggaaactta 60
   gcccatgcag 70
<210> 78
   <211> 70
   <212> DNA
   <213> Artificial Sequence
<220>
   <223> Description of Artificial Sequence: Synthetic probe
<400> 78
   atagtaaaag ccggggcggg tactggatga accgtatacc cacctttagc tggaaactta 60
   gcccatgcag 70
<210> 79
   <211> 70
   <212> DNA
   <213> Artificial Sequence
<220>
   <223> Description of Artificial Sequence: Synthetic probe
<400> 79
   atagtagaag ccggagcggg tactggatga actgtatatc cacctttagc tggaaacttg 60
   gcccatgcag 70
<210> 80
   <211> 60
   <212> DNA
   <213> Artificial Sequence
<220>
   <223> Description of Artificial Sequence: Synthetic probe
<400> 80
   gcccatgcag gagcctcagt agatctaact attttctccc tacatctggc aggtgtctct 60
<210> 81
   <211> 60
   <212> DNA
   <213> Artificial Sequence
<220>
   <223> Description of Artificial Sequence: Synthetic probe
<400> 81
   gcccatgcag gagcctcagt agacctaact attttctccc tacacctggc aggtgtctct 60
<210> 82
   <211> 60
   <212> DNA
   <213> Artificial Sequence
<220>
   <223> Description of Artificial Sequence: Synthetic probe
<400> 82
   gcccatgcag gagcctcagt agatctaatt attttctccc tacacctggc aggtgtctct 60
<210> 83
   <211> 60
   <212> DNA
   <213> Artificial Sequence
<220>
   <223> Description of Artificial Sequence: Synthetic probe
<400> 83
   gcccatgcag gagcctcagt agatctaact attttctccc tacacctggc aggtgtctct 60
<210> 84
   <211> 60
   <212> DNA
   <213> Artificial Sequence
<220>
   <223> Description of Artificial Sequence: Synthetic probe
<400> 84
   agcccatgca ggagcctcag tagatctaac tattttctcc ctacatctgg caggtgtctc 60
<210> 85
   <211> 60
   <212> DNA
   <213> Artificial Sequence
<220>
   <223> Description of Artificial Sequence: Synthetic probe
<400> 85
   agcccatgca ggagcctcag tagacctaac tattttctcc ctacacctgg caggtgtctc 60
<210> 86
   <211> 60
   <212> DNA
   <213> Artificial Sequence
<220>
   <223> Description of Artificial Sequence: Synthetic probe
<400> 86
   agcccatgca ggagcctcag tagatctaat tattttctcc ctacacctgg caggtgtctc 60
<210> 87
   <211> 60
   <212> DNA
   <213> Artificial Sequence
<220>
   <223> Description of Artificial Sequence: Synthetic probe
<400> 87
   agcccatgca ggagcctcag tagatctaac tattttctcc ctacacctgg caggtgtctc 60
<210> 88
   <211> 60
   <212> DNA
   <213> Artificial Sequence
<220>
   <223> Description of Artificial Sequence: Synthetic probe
<400> 88
   ctagcccatg caggagcctc agtagatcta actattttct ccctacatct ggcaggtgtc 60
<210> 89
   <211> 60
   <212> DNA
   <213> Artificial Sequence
<220>
   <223> Description of Artificial Sequence: Synthetic probe
<400> 89
   ctagcccatg caggagcctc agtagaccta actattttct ccctacacct ggcaggtgtc 60
<210> 90
   <211> 60
   <212> DNA
   <213> Artificial Sequence
<220>
   <223> Description of Artificial Sequence: Synthetic probe
<400> 90
   ctagcccatg caggagcctc agtagatcta attattttct ccctacacct ggcaggtgtc 60
<210> 91
   <211> 60
   <212> DNA
   <213> Artificial Sequence
<220>
   <223> Description of Artificial Sequence: Synthetic probe
<400> 91
   ctagcccatg caggagcctc agtagatcta actattttct ccctacacct ggcaggtgtc 60
<210> 92
   <211> 60
   <212> DNA
   <213> Artificial Sequence
<220>
   <223> Description of Artificial Sequence: Synthetic probe
<400> 92
   cctagcccat gcaggagcct cagtagatct aactattttc tccctacatc tggcaggtgt 60
<210> 93
   <211> 60
   <212> DNA
   <213> Artificial Sequence
<220>
   <223> Description of Artificial Sequence: Synthetic probe
<400> 93
   cctagcccat gcaggagcct cagtagacct aactattttc tccctacacc tggcaggtgt 60
<210> 94
   <211> 60
   <212> DNA
   <213> Artificial Sequence
<220>
   <223> Description of Artificial Sequence: Synthetic probe
<400> 94
   cctagcccat gcaggagcct cagtagatct aattattttc tccctacacc tggcaggtgt 60
<210> 95
   <211> 60
   <212> DNA
   <213> Artificial Sequence
<220>
   <223> Description of Artificial Sequence: Synthetic probe
<400> 95
   cctagcccat gcaggagcct cagtagatct aactattttc tccctacacc tggcaggtgt 60
<210> 96
   <211> 70
   <212> DNA
   <213> Artificial Sequence
<220>
   <223> Description of Artificial Sequence: Synthetic probe
<400> 96
   gcaacctagc ccatgcagga gcctcagtag atctaactat tttctcccta catctggcag 60
gtgtctcttc 70
<210> 97
   <211> 70
   <212> DNA
   <213> Artificial Sequence
<220>
   <223> Description of Artificial Sequence: Synthetic probe
<400> 97
   gcaacctagc ccatgcagga gcctcagtag atctaactat tttctcccta cacctggcag 60
   gtgtctcttc 70
<210> 98
   <211> 70
   <212> DNA
   <213> Artificial Sequence
<220>
   <223> Description of Artificial Sequence: Synthetic probe
<400> 98
   gcaacctagc ccatgcagga gcctcagtag atctaattat tttctcccta cacctggcag 60
   gtgtctcttc 70
<210> 99
   <211> 70
   <212> DNA
   <213> Artificial Sequence
<220>
   <223> Description of Artificial Sequence: Synthetic probe
<400> 99
   gcaacctagc ccatgcagga gcctcagtag acctaactat tttctcccta cacctggcag 60
   gtgtctcttc 70
<210> 100
   <211> 70
   <212> DNA
   <213> Artificial Sequence
<220>
   <223> Description of Artificial Sequence: Synthetic probe
<400> 100
   ggcaacctag cccatgcagg agcctcagta gatctaacta ttttctccct acatctggca 60
   ggtgtctctt 70
<210> 101
   <211> 70
   <212> DNA
   <213> Artificial Sequence
<220>
   <223> Description of Artificial Sequence: Synthetic probe
<400> 101
   ggcaacctag cccatgcagg agcctcagta gatctaacta ttttctccct acacctggca 60
   ggtgtctctt 70
<210> 102
   <211> 70
   <212> DNA
   <213> Artificial Sequence
<220>
   <223> Description of Artificial Sequence: Synthetic probe
<400> 102
   ggcaacctag cccatgcagg agcctcagta gatctaatta ttttctccct acacctggca 60
   ggtgtctctt 70
<210> 103
   <211> 70
   <212> DNA
   <213> Artificial Sequence
<220>
   <223> Description of Artificial Sequence: Synthetic probe
<400> 103
   ggcaacctag cccatgcagg agcctcagta gacctaacta ttttctccct acacctggca 60
   ggtgtctctt 70
<210> 104
   <211> 60
   <212> DNA
   <213> Artificial Sequence
<220>
   <223> Description of Artificial Sequence: Synthetic probe
<400> 104
   tgaaccgtat atcctcctct agctggaaat ctggcgcatg caggagcctc tgttgactta 60
<210> 105
   <211> 60
   <212> DNA
   <213> Artificial Sequence
<220>
   <223> Description of Artificial Sequence: Synthetic probe
<400> 105
   gaaccgtata tcctcctcta gctggaaatc tggcgcatgc aggagcctct gttgacttaa 60
<210> 106
   <211> 60
   <212> DNA
   <213> Artificial Sequence
<220>
   <223> Description of Artificial Sequence: Synthetic probe
<400> 106
   ctgaaccgta tatcctcctc tagctggaaa tctggcgcat gcaggagcct ctgttgactt 60
<210> 107
   <211> 60
   <212> DNA
   <213> Artificial Sequence
<220>
   <223> Description of Artificial Sequence: Synthetic probe
<400> 107
   aaccgtatat cctcctctag ctggaaatct ggcgcatgca ggagcctctg ttgacttaac 60
<210> 108
   <211> 60
   <212> DNA
   <213> Artificial Sequence
<220>
   <223> Description of Artificial Sequence: Synthetic probe
<400> 108
   cctctagctg gaaatctggc gcatgcagga gcctctgttg acttaaccat tttctctctc 60
<210> 109
   <211> 60
   <212> DNA
   <213> Artificial Sequence
<220>
   <223> Description of Artificial Sequence: Synthetic probe
<400> 109
   ctctagctgg aaatctggcg catgcaggag cctctgttga cttaaccatt ttctctctcc 60
<210> 110
   <211> 60
   <212> DNA
   <213> Artificial Sequence
<220>
   <223> Description of Artificial Sequence: Synthetic probe
<400> 110
   ctagctggaa atctggcgca tgcaggagcc tctgttgact taaccatttt ctctctccac 60
<210> 111
   <211> 60
   <212> DNA
   <213> Artificial Sequence
<220>
   <223> Description of Artificial Sequence: Synthetic probe
<400> 111
   gctggaaatc tggcgcatgc aggagcctct gttgacttaa ccattttctc tctccaccta 60
<210> 112
   <211> 60
   <212> DNA
   <213> Artificial Sequence
<220>
   <223> Description of Artificial Sequence: Synthetic probe
<400> 112
   gctggaaatc tggcgcatgc aggagcctct gttgacttaa ccattttctc tctccacctg 60
<210> 113
   <211> 60
   <212> DNA
   <213> Artificial Sequence
<220>
   <223> Description of Artificial Sequence: Synthetic probe
<400> 113
   ctggaaatct ggcgcatgca ggagcctctg ttgacttaac cattttctct ctccacctag 60
<210> 114
   <211> 60
   <212> DNA
   <213> Artificial Sequence
<220>
   <223> Description of Artificial Sequence: Synthetic probe
<400> 114
   ctggaaatct ggcgcatgca ggagcctctg ttgacttaac cattttctct ctccacctgg 60
<210> 115
   <211> 60
   <212> DNA
   <213> Artificial Sequence
<220>
   <223> Description of Artificial Sequence: Synthetic probe
<400> 115
   tggaaatctg gcgcatgcag gagcctctgt tgacttaacc attttctctc tccacctagc 60
<210> 116
   <211> 60
   <212> DNA
   <213> Artificial Sequence
<220>
   <223> Description of Artificial Sequence: Synthetic probe
<400> 116
   tggaaatctg gcgcatgcag gagcctctgt tgacttaacc attttctctc tccacctggc 60
<210> 117
   <211> 60
   <212> DNA
   <213> Artificial Sequence
<220>
   <223> Description of Artificial Sequence: Synthetic probe
<400> 117
   ggaaatctgg cgcatgcagg agcctctgtt gacttaacca ttttctctct ccacctagct 60
<210> 118
   <211> 60
   <212> DNA
   <213> Artificial Sequence
<220>
   <223> Description of Artificial Sequence: Synthetic probe
<400> 118
   ggaaatctgg cgcatgcagg agcctctgtt gacttaacca ttttctctct ccacctggct 60
<210> 119
   <211> 60
   <212> DNA
   <213> Artificial Sequence
<220>
   <223> Description of Artificial Sequence: Synthetic probe
<400> 119
   gaaatctggc gcatgcagga gcctctgttg acttaaccat tttctctctc cacctagctg 60
<210> 120
   <211> 60
   <212> DNA
   <213> Artificial Sequence
<220>
   <223> Description of Artificial Sequence: Synthetic probe
<400> 120
   gaaatctggc gcatgcagga gcctctgttg acttaaccat tttctctctc cacctggctg 60
<210> 121
   <211> 60
   <212> DNA
   <213> Artificial Sequence
<220>
   <223> Description of Artificial Sequence: Synthetic probe
<400> 121
   tcctcctcta gctggaaatc tggcgcatgc aggagcctct gttgacttaa ccattttctc 60
<210> 122
   <211> 60
   <212> DNA
   <213> Artificial Sequence
<220>
   <223> Description of Artificial Sequence: Synthetic probe
<400> 122
   cctcctctag ctggaaatct ggcgcatgca ggagcctctg ttgacttaac cattttctct 60
<210> 123
   <211> 60
   <212> DNA
   <213> Artificial Sequence
<220>
   <223> Description of Artificial Sequence: Synthetic probe
<400> 123
   ctcctctagc tggaaatctg gcgcatgcag gagcctctgt tgacttaacc attttctctc 60
<210> 124
   <211> 60
   <212> DNA
   <213> Artificial Sequence
<220>
   <223> Description of Artificial Sequence: Synthetic probe
<400> 124
   cgtatatcct cctctagctg gaaatctggc gcatgcagga gcctctgttg acttaaccat 60
<210> 125
   <211> 60
   <212> DNA
   <213> Artificial Sequence
<220>
   <223> Description of Artificial Sequence: Synthetic probe
<400> 125
   accgtatatc ctcctctagc tggaaatctg gcgcatgcag gagcctctgt tgacttaacc 60
<210> 126
   <211> 60
   <212> DNA
   <213> Artificial Sequence
<220>
   <223> Description of Artificial Sequence: Synthetic probe
<400> 126
   ccgtatatcc tcctctagct ggaaatctgg cgcatgcagg agcctctgtt gacttaacca 60
<210> 127
   <211> 70
   <212> DNA
   <213> Artificial Sequence
<220>
   <223> Description of Artificial Sequence: Synthetic probe
<400> 127
   tatcctcctc tagctggaaa tctggcgcat gcaggagcct ctgttgactt aaccattttc 60
   tctctccacc 70
<210> 128
   <211> 70
   <212> DNA
   <213> Artificial Sequence
<220>
   <223> Description of Artificial Sequence: Synthetic probe
<400> 128
   atcctcctct agctggaaat ctggcgcatg caggagcctc tgttgactta accattttct 60
   ctctccacct 70
<210> 129
   <211> 70
   <212> DNA
   <213> Artificial Sequence
<220>
   <223> Description of Artificial Sequence: Synthetic probe
<400> 129
   ggctgaaccg tatatcctcc tctagctgga aatctggcgc atgcaggagc ctctgttgac 60
   ttaaccattt 70
<210> 130
   <211> 70
   <212> DNA
   <213> Artificial Sequence
<220>
   <223> Description of Artificial Sequence: Synthetic probe
<400> 130
   aggctgaacc gtatatcctc ctctagctgg aaatctggcg catgcaggag cctctgttga 60
   cttaaccatt 70
<210> 131
   <211> 70
   <212> DNA
   <213> Artificial Sequence
<220>
   <223> Description of Artificial Sequence: Synthetic probe
<400> 131
   cctctagctg gaaatctggc gcatgcagga gcctctgttg acttaaccat tttctctctc 60
   cacctagctg 70
<210> 132
   <211> 70
   <212> DNA
   <213> Artificial Sequence
<220>
   <223> Description of Artificial Sequence: Synthetic probe
<400> 132
   cctctagctg gaaatctggc gcatgcagga gcctctgttg acttaaccat tttctctctc 60
   cacctggctg 70
<210> 133
   <211> 70
   <212> DNA
   <213> Artificial Sequence
<220>
   <223> Description of Artificial Sequence: Synthetic probe
<400> 133
   tcctcctcta gctggaaatc tggcgcatgc aggagcctct gttgacttaa ccattttctc 60
   tctccaccta 70
<210> 134
   <211> 70
   <212> DNA
   <213> Artificial Sequence
<220>
   <223> Description of Artificial Sequence: Synthetic probe
<400> 134
   tcctcctcta gctggaaatc tggcgcatgc aggagcctct gttgacttaa ccattttctc 60
   tctccacctg 70
<210> 135
   <211> 70
   <212> DNA
   <213> Artificial Sequence
<220>
   <223> Description of Artificial Sequence: Synthetic probe
<400> 135
   tcctctagct ggaaatctgg cgcatgcagg agcctctgtt gacttaacca ttttctctct 60
   ccacctagct 70
<210> 136
   <211> 70
   <212> DNA
   <213> Artificial Sequence
<220>
   <223> Description of Artificial Sequence: Synthetic probe
<400> 136
   tcctctagct ggaaatctgg cgcatgcagg agcctctgtt gacttaacca ttttctctct 60
   ccacctggct 70
<210> 137
   <211> 70
   <212> DNA
   <213> Artificial Sequence
<220>
   <223> Description of Artificial Sequence: Synthetic probe
<400> 137
   cctcctctag ctggaaatct ggcgcatgca ggagcctctg ttgacttaac cattttctct 60
   ctccacctag 70
<210> 138
   <211> 70
   <212> DNA
   <213> Artificial Sequence
<220>
   <223> Description of Artificial Sequence: Synthetic probe
<400> 138
   cctcctctag ctggaaatct ggcgcatgca ggagcctctg ttgacttaac cattttctct 60
   ctccacctgg 70
<210> 139
   <211> 70
   <212> DNA
   <213> Artificial Sequence
<220>
   <223> Description of Artificial Sequence: Synthetic probe
<400> 139
   ctcctctagc tggaaatctg gcgcatgcag gagcctctgt tgacttaacc attttctctc 60
   tccacctagc 70
<210> 140
   <211> 70
   <212> DNA
   <213> Artificial Sequence
<220>
   <223> Description of Artificial Sequence: Synthetic probe
<400> 140
   ctcctctagc tggaaatctg gcgcatgcag gagcctctgt tgacttaacc attttctctc 60
   tccacctggc 70
<210> 141
   <211> 60
   <212> DNA
   <213> Artificial Sequence
<220>
   <223> Description of Artificial Sequence: Synthetic probe
<400> 141
   cctcctcatc cgagccgaac taggtcagcc cggtacttta ctaggtgacg atcaaattta 60
<210> 142
   <211> 60
   <212> DNA
   <213> Artificial Sequence
<220>
   <223> Description of Artificial Sequence: Synthetic probe
<400> 142
   cctcctcatc cgagccgaac taggtcagcc cggtacttta ctaggtgatg atcaaattta 60
<210> 143
   <211> 60
   <212> DNA
   <213> Artificial Sequence
<220>
   <223> Description of Artificial Sequence: Synthetic probe
<400> 143
   cctcctcatc cgagccgaac taggtcagcc cggtacttta ctaggcgacg atcaaattta 60
<210> 144
   <211> 60
   <212> DNA
   <213> Artificial Sequence
<220>
   <223> Description of Artificial Sequence: Synthetic probe
<400> 144
   cctcctcatc cgggccgaac taggtcagcc cggtacttta ctaggtgacg atcaaattta 60
<210> 145
   <211> 60
   <212> DNA
   <213> Artificial Sequence
<220>
   <223> Description of Artificial Sequence: Synthetic probe
<400> 145
   cctcctcatc cgagccgaac taggtcagcc cggtacttta ctaggagacg atcagattta 60
<210> 146
   <211> 60
   <212> DNA
   <213> Artificial Sequence
<220>
   <223> Description of Artificial Sequence: Synthetic probe
<400> 146
   agcctcctca tccgagccga actaggtcag cccggtactt tactaggtga cgatcaaatt 60
<210> 147
   <211> 60
   <212> DNA
   <213> Artificial Sequence
<220>
   <223> Description of Artificial Sequence: Synthetic probe
<400> 147
   agcctcctca tccgagccga actaggtcag cccggtactt tactaggtga tgatcaaatt 60
<210> 148
   <211> 60
   <212> DNA
   <213> Artificial Sequence
<220>
   <223> Description of Artificial Sequence: Synthetic probe
<400> 148
   agcctcctca tccgagccga actaggtcag cccggtactt tactaggcga cgatcaaatt 60
<210> 149
   <211> 60
   <212> DNA
   <213> Artificial Sequence
<220>
   <223> Description of Artificial Sequence: Synthetic probe
<400> 149
   agcctcctca tccgggccga actaggtcag cccggtactt tactaggtga cgatcaaatt 60
<210> 150
   <211> 60
   <212> DNA
   <213> Artificial Sequence
<220>
   <223> Description of Artificial Sequence: Synthetic probe
<400> 150
   agcctcctca tccgagccga actaggtcag cccggtactt tactaggaga cgatcagatt 60
<210> 151
   <211> 60
   <212> DNA
   <213> Artificial Sequence
<220>
   <223> Description of Artificial Sequence: Synthetic probe
<400> 151
   gcctcctcat ccgagccgaa ctaggtcagc ccggtacttt actaggtgac gatcaaattt 60
<210> 152
   <211> 60
   <212> DNA
   <213> Artificial Sequence
<220>
   <223> Description of Artificial Sequence: Synthetic probe
<400> 152
   gcctcctcat ccgagccgaa ctaggtcagc ccggtacttt actaggtgat gatcaaattt 60
<210> 153
   <211> 60
   <212> DNA
   <213> Artificial Sequence
<220>
   <223> Description of Artificial Sequence: Synthetic probe
<400> 153
   gcctcctcat ccgagccgaa ctaggtcagc ccggtacttt actaggcgac gatcaaattt 60
<210> 154
   <211> 60
   <212> DNA
   <213> Artificial Sequence
<220>
   <223> Description of Artificial Sequence: Synthetic probe
<400> 154
   gcctcctcat ccgggccgaa ctaggtcagc ccggtacttt actaggtgac gatcaaattt 60
<210> 155
   <211> 70
   <212> DNA
   <213> Artificial Sequence
<220>
   <223> Description of Artificial Sequence: Synthetic probe
<400> 155
   cactgctttg agcctcctca tccgagccga actaggtcag cccggtactt tactaggtga 60
   cgatcaaatt 70
<210> 156
   <211> 70
   <212> DNA
   <213> Artificial Sequence
<220>
   <223> Description of Artificial Sequence: Synthetic probe
<400> 156
   cactgccttg agcctcctca tccgagccga actaggtcag cccggtactt tactaggtga 60
   cgatcaaatt 70
<210> 157
   <211> 70
   <212> DNA
   <213> Artificial Sequence
<220>
   <223> Description of Artificial Sequence: Synthetic probe
<400> 157
   cactgccttg agcctcctca tccgagccga actaggtcag cccggtactt tactaggtga 60
   tgatcaaatt 70
<210> 158
   <211> 70
   <212> DNA
   <213> Artificial Sequence
<220>
   <223> Description of Artificial Sequence: Synthetic probe
<400> 158
   cactgccttg agcctcctca tccgagccga actaggtcag cccggtactt tactaggcga 60
   cgatcaaatt 70
<210> 159
   <211> 70
   <212> DNA
   <213> Artificial Sequence
<220>
   <223> Description of Artificial Sequence: Synthetic probe
<400> 159
   cactgccttg agcctcctca tccgagccga gctaggtcag cccggtactt tactaggcga 60
   cgaccaaatt 70
<210> 160
   <211> 70
   <212> DNA
   <213> Artificial Sequence
<220>
   <223> Description of Artificial Sequence: Synthetic probe
<400> 160
   ctgctttgag cctcctcatc cgagccgaac taggtcagcc cggtacttta ctaggtgacg 60
   atcaaattta 70
<210> 161
   <211> 70
   <212> DNA
   <213> Artificial Sequence
<220>
   <223> Description of Artificial Sequence: Synthetic probe
<400> 161
   ctgccttgag cctcctcatc cgagccgaac taggtcagcc cggtacttta ctaggtgacg 60
   atcaaattta 70
<210> 162
   <211> 70
   <212> DNA
   <213> Artificial Sequence
<220>
   <223> Description of Artificial Sequence: Synthetic probe
<400> 162
   ctgccttgag cctcctcatc cgagccgaac taggtcagcc cggtacttta ctaggtgatg 60
   atcaaattta 70
<210> 163
   <211> 70
   <212> DNA
   <213> Artificial Sequence
<220>
   <223> Description of Artificial Sequence: Synthetic probe
<400> 163
   ctgccttgag cctcctcatc cgagccgaac taggtcagcc cggtacttta ctaggcgacg 60
   atcaaattta 70
<210> 164
   <211> 70
   <212> DNA
   <213> Artificial Sequence
<220>
   <223> Description of Artificial Sequence: Synthetic probe
<400> 164
   ctgccttgag cctcctcatc cgagccgagc taggtcagcc cggtacttta ctaggcgacg 60
   accaaattta 70
<210> 165
   <211> 70
   <212> DNA
   <213> Artificial Sequence
<220>
   <223> Description of Artificial Sequence: Synthetic probe
<400> 165
   actgctttga gcctcctcat ccgagccgaa ctaggtcagc ccggtacttt actaggtgac 60
   gatcaaattt 70
<210> 166
   <211> 70
   <212> DNA
   <213> Artificial Sequence
<220>
   <223> Description of Artificial Sequence: Synthetic probe
<400> 166
   actgccttga gcctcctcat ccgagccgaa ctaggtcagc ccggtacttt actaggtgac 60
   gatcaaattt 70
<210> 167
   <211> 70
   <212> DNA
   <213> Artificial Sequence
<220>
   <223> Description of Artificial Sequence: Synthetic probe
<400> 167
   actgccttga gcctcctcat ccgagccgaa ctaggtcagc ccggtacttt actaggtgat 60
   gatcaaattt 70
<210> 168
   <211> 70
   <212> DNA
   <213> Artificial Sequence
<220>
   <223> Description of Artificial Sequence: Synthetic probe
<400> 168
   actgccttga gcctcctcat ccgagccgaa ctaggtcagc ccggtacttt actaggcgac 60
   gatcaaattt 70
<210> 169
   <211> 70
   <212> DNA
   <213> Artificial Sequence
<220>
   <223> Description of Artificial Sequence: Synthetic probe
<400> 169
   actgccttga gcctcctcat ccgagccgag ctaggtcagc ccggtacttt actaggcgac 60
   gaccaaattt 70
<210> 170
   <211> 60
   <212> DNA
   <213> Artificial Sequence
<220>
   <223> Description of Artificial Sequence: Synthetic probe
<400> 170
   caatagtaga agcaggagca ggaacaggat gaacagtcta cccacctcta gccggaaatc 60
<210> 171
   <211> 60
   <212> DNA
   <213> Artificial Sequence
<220>
   <223> Description of Artificial Sequence: Synthetic probe
<400> 171
   tagtagaagc aggagcagga acaggatgaa cagtctaccc acctctagcc ggaaatctag 60
<210> 172
   <211> 60
   <212> DNA
   <213> Artificial Sequence
<220>
   <223> Description of Artificial Sequence: Synthetic probe
<400> 172
   tagtagaagc aggagcagga acaggatgaa cagtctaccc acctctagcc ggaaatccag 60
<210> 173
   <211> 60
   <212> DNA
   <213> Artificial Sequence
<220>
   <223> Description of Artificial Sequence: Synthetic probe
<400> 173
   agtagaagca ggagcaggaa caggatgaac agtctaccca cctctagccg gaaatctagc 60
<210> 174
   <211> 60
   <212> DNA
   <213> Artificial Sequence
<220>
   <223> Description of Artificial Sequence: Synthetic probe
<400> 174
   agtagaagca ggagcaggaa caggatgaac agtctaccca cctctagccg gaaatccagt 60
<210> 175
   <211> 60
   <212> DNA
   <213> Artificial Sequence
<220>
   <223> Description of Artificial Sequence: Synthetic probe
<400> 175
   gtctacccac ctctagccgg aaatctagcc catgcaggag catcagtaga cctaacaatt 60
<210> 176
   <211> 60
   <212> DNA
   <213> Artificial Sequence
<220>
   <223> Description of Artificial Sequence: Synthetic probe
<400> 176
   gtctacccac ctctagccgg aaatccagtc catgcaggag catcagtaga cctaacaatt 60
<210> 177
   <211> 60
   <212> DNA
   <213> Artificial Sequence
<220>
   <223> Description of Artificial Sequence: Synthetic probe
<400> 177
   gtctacccac ctctagccgg aaatctagcc catgcaggag catcagtaga tctaacaatt 60
<210> 178
   <211> 60
   <212> DNA
   <213> Artificial Sequence
<220>
   <223> Description of Artificial Sequence: Synthetic probe
<400> 178
   cccacctcta gccggaaatc tagcccatgc aggagcatca gtagacctaa caattttctc 60
<210> 179
   <211> 60
   <212> DNA
   <213> Artificial Sequence
<220>
   <223> Description of Artificial Sequence: Synthetic probe
<400> 179
   cccacctcta gccggaaatc cagtccatgc aggagcatca gtagacctaa caattttctc 60
<210> 180
   <211> 60
   <212> DNA
   <213> Artificial Sequence
<220>
   <223> Description of Artificial Sequence: Synthetic probe
<400> 180
   cccacctcta gccggaaatc tagcccatgc aggagcatca gtagatctaa caattttctc 60
<210> 181
   <211> 60
   <212> DNA
   <213> Artificial Sequence
<220>
   <223> Description of Artificial Sequence: Synthetic probe
<400> 181
   ccacctctag ccggaaatct agcccatgca ggagcatcag tagacctaac aattttctcc 60
<210> 182
   <211> 60
   <212> DNA
   <213> Artificial Sequence
<220>
   <223> Description of Artificial Sequence: Synthetic probe
<400> 182
   ccacctctag ccggaaatcc agtccatgca ggagcatcag tagacctaac aattttctcc 60
<210> 183
   <211> 60
   <212> DNA
   <213> Artificial Sequence
<220>
   <223> Description of Artificial Sequence: Synthetic probe
<400> 183
   ccacctctag ccggaaatct agcccatgca ggagcatcag tagatctaac aattttctcc 60
<210> 184
   <211> 60
   <212> DNA
   <213> Artificial Sequence
<220>
   <223> Description of Artificial Sequence: Synthetic probe
<400> 184
   aggatgaaca gtctacccac ctctagccgg aaatctagcc catgcaggag catcagtaga 60
<210> 185
   <211> 60
   <212> DNA
   <213> Artificial Sequence
<220>
   <223> Description of Artificial Sequence: Synthetic probe
<400> 185
   aggatgaaca gtctacccac ctctagccgg aaatccagtc catgcaggag catcagtaga 60
<210> 186
   <211> 60
   <212> DNA
   <213> Artificial Sequence
<220>
   <223> Description of Artificial Sequence: Synthetic probe
<400> 186
   aggatgaacc gtatatccac ctttagccgg aaatttagcc cacgccggag catcagtgga 60
<210> 187
   <211> 60
   <212> DNA
   <213> Artificial Sequence
<220>
   <223> Description of Artificial Sequence: Synthetic probe
<400> 187
   agtctaccca cctctagccg gaaatctagc ccatgcagga gcatcagtag acctaacaat 60
<210> 188
   <211> 60
   <212> DNA
   <213> Artificial Sequence
<220>
   <223> Description of Artificial Sequence: Synthetic probe
<400> 188
   agtctaccca cctctagccg gaaatccagt ccatgcagga gcatcagtag acctaacaat 60
<210> 189
   <211> 60
   <212> DNA
   <213> Artificial Sequence
<220>
   <223> Description of Artificial Sequence: Synthetic probe
<400> 189
   agtctaccca cctctagccg gaaatctagc ccatgcagga gcatcagtag atctaacaat 60
<210> 190
   <211> 60
   <212> DNA
   <213> Artificial Sequence
<220>
   <223> Description of Artificial Sequence: Synthetic probe
<400> 190
   atgaacagtc tacccacctc tagccggaaa tctagcccat gcaggagcat cagtagacct 60
<210> 191
   <211> 60
   <212> DNA
   <213> Artificial Sequence
<220>
   <223> Description of Artificial Sequence: Synthetic probe
<400> 191
   atgaacagtc tacccacctc tagccggaaa tccagtccat gcaggagcat cagtagacct 60
<210> 192
   <211> 60
   <212> DNA
   <213> Artificial Sequence
<220>
   <223> Description of Artificial Sequence: Synthetic probe
<400> 192
   atgaacagtc tacccacctc tagccggaaa tctagcccat gcaggagcat cagtagatct 60
<210> 193
   <211> 60
   <212> DNA
   <213> Artificial Sequence
<220>
   <223> Description of Artificial Sequence: Synthetic probe
<400> 193
   tgaacagtct acccacctct agccggaaat ctagcccatg caggagcatc agtagaccta 60
<210> 194
   <211> 60
   <212> DNA
   <213> Artificial Sequence
<220>
   <223> Description of Artificial Sequence: Synthetic probe
<400> 194
   tgaacagtct acccacctct agccggaaat ccagtccatg caggagcatc agtagaccta 60
<210> 195
   <211> 60
   <212> DNA
   <213> Artificial Sequence
<220>
   <223> Description of Artificial Sequence: Synthetic probe
<400> 195
   tgaacagtct acccacctct agccggaaat ctagcccatg caggagcatc agtagatcta 60
<210> 196
   <211> 60
   <212> DNA
   <213> Artificial Sequence
<220>
   <223> Description of Artificial Sequence: Synthetic probe
<400> 196
   gaacagtcta cccacctcta gccggaaatc tagcccatgc aggagcatca gtagacctaa 60
<210> 197
   <211> 60
   <212> DNA
   <213> Artificial Sequence
<220>
   <223> Description of Artificial Sequence: Synthetic probe
<400> 197
   gaacagtcta cccacctcta gccggaaatc cagtccatgc aggagcatca gtagacctaa 60
<210> 198
   <211> 60
   <212> DNA
   <213> Artificial Sequence
<220>
   <223> Description of Artificial Sequence: Synthetic probe
<400> 198
   gaacagtcta cccacctcta gccggaaatc tagcccatgc aggagcatca gtagatctaa 60
<210> 199
   <211> 60
   <212> DNA
   <213> Artificial Sequence
<220>
   <223> Description of Artificial Sequence: Synthetic probe
<400> 199
   aacagtctac ccacctctag ccggaaatct agcccatgca ggagcatcag tagacctaac 60
<210> 200
   <211> 60
   <212> DNA
   <213> Artificial Sequence
<220>
   <223> Description of Artificial Sequence: Synthetic probe
<400> 200
   aacagtctac ccacctctag ccggaaatcc agtccatgca ggagcatcag tagacctaac 60
<210> 201
   <211> 60
   <212> DNA
   <213> Artificial Sequence
<220>
   <223> Description of Artificial Sequence: Synthetic probe
<400> 201
   aacagtctac ccacctctag ccggaaatct agcccatgca ggagcatcag tagatctaac 60
<210> 202
   <211> 60
   <212> DNA
   <213> Artificial Sequence
<220>
   <223> Description of Artificial Sequence: Synthetic probe
<400> 202
   acagtctacc cacctctagc cggaaatcta gcccatgcag gagcatcagt agacctaaca 60
<210> 203
   <211> 60
   <212> DNA
   <213> Artificial Sequence
<220>
   <223> Description of Artificial Sequence: Synthetic probe
<400> 203
   acagtctacc cacctctagc cggaaatcca gtccatgcag gagcatcagt agacctaaca 60
<210> 204
   <211> 60
   <212> DNA
   <213> Artificial Sequence
<220>
   <223> Description of Artificial Sequence: Synthetic probe
<400> 204
   acagtctacc cacctctagc cggaaatcta gcccatgcag gagcatcagt agatctaaca 60
<210> 205
   <211> 60
   <212> DNA
   <213> Artificial Sequence
<220>
   <223> Description of Artificial Sequence: Synthetic probe
<400> 205
   cagtctaccc acctctagcc ggaaatctag cccatgcagg agcatcagta gacctaacaa 60
<210> 206
   <211> 60
   <212> DNA
   <213> Artificial Sequence
<220>
   <223> Description of Artificial Sequence: Synthetic probe
<400> 206
   cagtctaccc acctctagcc ggaaatccag tccatgcagg agcatcagta gacctaacaa 60
<210> 207
   <211> 60
   <212> DNA
   <213> Artificial Sequence
<220>
   <223> Description of Artificial Sequence: Synthetic probe
<400> 207
   cagtctaccc acctctagcc ggaaatctag cccatgcagg agcatcagta gatctaacaa 60
<210> 208
   <211> 70
   <212> DNA
   <213> Artificial Sequence
<220>
   <223> Description of Artificial Sequence: Synthetic probe
<400> 208
   tcaatagtag aagcaggagc aggaacagga tgaacagtct acccacctct agccggaaat 60
   ctagcccatg 70
<210> 209
   <211> 70
   <212> DNA
   <213> Artificial Sequence
<220>
   <223> Description of Artificial Sequence: Synthetic probe
<400> 209
   tcaatagtag aagcaggagc aggaacagga tgaacagtct acccacctct agccggaaat 60
   ccagtccatg 70
<210> 210
   <211> 70
   <212> DNA
   <213> Artificial Sequence
<220>
   <223> Description of Artificial Sequence: Synthetic probe
<400> 210
   caatagtaga agcaggagca ggaacaggat gaacagtcta cccacctcta gccggaaatc 60
   tagcccatgc 70
<210> 211
   <211> 70
   <212> DNA
   <213> Artificial Sequence
<220>
   <223> Description of Artificial Sequence: Synthetic probe
<400> 211
   caatagtaga agcaggagca ggaacaggat gaacagtcta cccacctcta gccggaaatc 60
   cagtccatgc 70
<210> 212
   <211> 70
   <212> DNA
   <213> Artificial Sequence
<220>
   <223> Description of Artificial Sequence: Synthetic probe
<400> 212
   gtctacccac ctctagccgg aaatctagcc catgcaggag catcagtaga cctaacaatt 60
   ttctcccttc 70
<210> 213
   <211> 70
   <212> DNA
   <213> Artificial Sequence
<220>
   <223> Description of Artificial Sequence: Synthetic probe
<400> 213
   gtctacccac ctctagccgg aaatctagcc catgcaggag catcagtaga cctaacaatt 60
   ttctccctcc 70
<210> 214
   <211> 70
   <212> DNA
   <213> Artificial Sequence
<220>
   <223> Description of Artificial Sequence: Synthetic probe
<400> 214
   gtctacccac ctctagccgg aaatctagcc catgcaggag catcagtaga tctaacaatt 60
   ttctcccttc 70
<210> 215
   <211> 70
   <212> DNA
   <213> Artificial Sequence
<220>
   <223> Description of Artificial Sequence: Synthetic probe
<400> 215
   gtctacccac ctctagccgg aaatccagtc catgcaggag catcagtaga cctaacaatt 60
   ttctcccttc 70
<210> 216
   <211> 70
   <212> DNA
   <213> Artificial Sequence
<220>
   <223> Description of Artificial Sequence: Synthetic probe
<400> 216
   caggatgaac agtctaccca cctctagccg gaaatctagc ccatgcagga gcatcagtag 60
   acctaacaat 70
<210> 217
   <211> 70
   <212> DNA
   <213> Artificial Sequence
<220>
   <223> Description of Artificial Sequence: Synthetic probe
<400> 217
   caggatgaac agtctaccca cctctagccg gaaatctagc ccatgcagga gcatcagtag 60
   atctaacaat 70
<210> 218
   <211> 70
   <212> DNA
   <213> Artificial Sequence
<220>
   <223> Description of Artificial Sequence: Synthetic probe
<400> 218
   caggatgaac agtctaccca cctctagccg gaaatccagt ccatgcagga gcatcagtag 60
   acctaacaat 70
<210> 219
   <211> 60
   <212> DNA
   <213> Artificial Sequence
<220>
   <223> Description of Artificial Sequence: Synthetic probe
<400> 219
   gagcttctga ctcctccctc catcctttct actcttactc gcctcatcta tggtagaagc 60
<210> 220
   <211> 60
   <212> DNA
   <213> Artificial Sequence
<220>
   <223> Description of Artificial Sequence: Synthetic probe
<400> 220
   gagcttctga ctcctccccc catcctttct actcttactc gcctcatcta tggtagaagc 60
<210> 221
   <211> 60
   <212> DNA
   <213> Artificial Sequence
<220>
   <223> Description of Artificial Sequence: Synthetic probe
<400> 221
   gagcttctga ctcctccctc catcctttct actcctactc gcctcatcta tggtagaagc 60
<210> 222
   <211> 60
   <212> DNA
   <213> Artificial Sequence
<220>
   <223> Description of Artificial Sequence: Synthetic probe
<400> 222
   ctccctccat cctttctact cttactcgcc tcatctatgg tagaagccgg agcaggaact 60
<210> 223
   <211> 60
   <212> DNA
   <213> Artificial Sequence
<220>
   <223> Description of Artificial Sequence: Synthetic probe
<400> 223
   ctccccccat cctttctact cttactcgcc tcatctatgg tagaagccgg agcaggaact 60
<210> 224
   <211> 60
   <212> DNA
   <213> Artificial Sequence
<220>
   <223> Description of Artificial Sequence: Synthetic probe
<400> 224
   ctccctccat cctttctact cctactcgcc tcatctatgg tagaagccgg agcaggaact 60
<210> 225
   <211> 60
   <212> DNA
   <213> Artificial Sequence
<220>
   <223> Description of Artificial Sequence: Synthetic probe
<400> 225
   tcctccctcc atcctttcta ctcttactcg cctcatctat ggtagaagcc ggagcaggaa 60
<210> 226
   <211> 60
   <212> DNA
   <213> Artificial Sequence
<220>
   <223> Description of Artificial Sequence: Synthetic probe
<400> 226
   tcctcccccc atcctttcta ctcttactcg cctcatctat ggtagaagcc ggagcaggaa 60
<210> 227
   <211> 60
   <212> DNA
   <213> Artificial Sequence
<220>
   <223> Description of Artificial Sequence: Synthetic probe
<400> 227
   tcctccctcc atcctttcta ctcctactcg cctcatctat ggtagaagcc ggagcaggaa 60
<210> 228
   <211> 60
   <212> DNA
   <213> Artificial Sequence
<220>
   <223> Description of Artificial Sequence: Synthetic probe
<400> 228
   tgactcctcc ctccatcctt tctactctta ctcgcctcat ctatggtaga agccggagca 60
<210> 229
   <211> 60
   <212> DNA
   <213> Artificial Sequence
<220>
   <223> Description of Artificial Sequence: Synthetic probe
<400> 229
   tgactcctcc ccccatcctt tctactctta ctcgcctcat ctatggtaga agccggagca 60
<210> 230
   <211> 60
   <212> DNA
   <213> Artificial Sequence
<220>
   <223> Description of Artificial Sequence: Synthetic probe
<400> 230
   tgactcctcc ctccatcctt tctactccta ctcgcctcat ctatggtaga agccggagca 60
<210> 231
   <211> 60
   <212> DNA
   <213> Artificial Sequence
<220>
   <223> Description of Artificial Sequence: Synthetic probe
<400> 231
   gctcctgaca tagcatttcc ccgaataaac aacatgagct tctgactcct ccctccatcc 60
<210> 232
   <211> 60
   <212> DNA
   <213> Artificial Sequence
<220>
   <223> Description of Artificial Sequence: Synthetic probe
<400> 232
   gctcctgaca tagcatttcc ccgaataaac aacatgagct tctgactcct ccccccatcc 60
<210> 233
   <211> 70
   <212> DNA
   <213> Artificial Sequence
<220>
   <223> Description of Artificial Sequence: Synthetic probe
<400> 233
   atgagcttct gactcctccc tccatccttt ctactcttac tcgcctcatc tatggtagaa 60
   gccggagcag 70
<210> 234
   <211> 70
   <212> DNA
   <213> Artificial Sequence
<220>
   <223> Description of Artificial Sequence: Synthetic probe
<400> 234
   atgagcttct gactcccccc cccatccttt ctactcttac tcgcctcatc tatggtagaa 60
   gccggagcag 70
<210> 235
   <211> 70
   <212> DNA
   <213> Artificial Sequence
<220>
   <223> Description of Artificial Sequence: Synthetic probe
<400> 235
   atgagcttct gactcctccc tccatccttt ctactcctac tcgcctcatc tatggtagaa 60
   gccggagcag 70
<210> 236
   <211> 70
   <212> DNA
   <213> Artificial Sequence
<220>
   <223> Description of Artificial Sequence: Synthetic probe
<400> 236
   agcttctgac tcctccctcc atcctttcta ctcttactcg cctcatctat ggtagaagcc 60
   ggagcaggaa 70
<210> 237
   <211> 70
   <212> DNA
   <213> Artificial Sequence
<220>
   <223> Description of Artificial Sequence: Synthetic probe
<400> 237
   agcttctgac tccccccccc atcctttcta ctcttactcg cctcatctat ggtagaagcc 60
   ggagcaggaa 70
<210> 238
   <211> 70
   <212> DNA
   <213> Artificial Sequence
<220>
   <223> Description of Artificial Sequence: Synthetic probe
<400> 238
   agcttctgac tcctccctcc atcctttcta ctcctactcg cctcatctat ggtagaagcc 60
   ggagcaggaa 70
<210> 239
   <211> 70
   <212> DNA
   <213> Artificial Sequence
<220>
   <223> Description of Artificial Sequence: Synthetic probe
<400> 239
   cttctgactc ctccctccat cctttctact cttactcgcc tcatctatgg tagaagccgg 60
   agcaggaact 70
<210> 240
   <211> 70
   <212> DNA
   <213> Artificial Sequence
<220>
   <223> Description of Artificial Sequence: Synthetic probe
<400> 240
   cttctgactc ccccccccat cctttctact cttactcgcc tcatctatgg tagaagccgg 60
   agcaggaact 70
<210> 241
   <211> 70
   <212> DNA
   <213> Artificial Sequence
<220>
   <223> Description of Artificial Sequence: Synthetic probe
<400> 241
   cttctgactc ctccctccat cctttctact cctactcgcc tcatctatgg tagaagccgg      60
   agcaggaact                                                             70